(19) Europäisches Patentamt
European Patent Office
Office européen des brevets

(11) Veröffentlichungsnummer: **0 256 985 A2**

(12) # EUROPÄISCHE PATENTANMELDUNG

(21) Anmeldenummer: **87810455.3**

(22) Anmeldetag: **10.08.87**

(51) Int.Cl.[3]: **C 07 D 237/14**
C 07 D 239/34, C 07 D 241/18
C 07 D 241/44, C 07 D 253/02
C 07 D 257/08, C 07 D 237/28
C 07 D 215/20, C 07 D 217/24
C 07 D 239/80

(30) Priorität: **15.08.86 CH 3295/86**

(43) Veröffentlichungstag der Anmeldung:
**24.02.88 Patentblatt 88/8**

(84) Benannte Vertragsstaaten:
**BE CH DE ES FR GB IT LI NL**

(71) Anmelder: **CIBA-GEIGY AG**
**Klybeckstrasse 141**
**CH-4002 Basel(CH)**

(72) Erfinder: **Böger, Manfred**
**Wilhelm Glockstrasse 14**
**D-7858 Weil am Rhein 5(DE)**

(72) Erfinder: **Drabek, Jozef, Dr.**
**Benkenstrasse 12**
**CH-4104 Oberwil(CH)**

(72) Erfinder: **Ehrenfreund, Josef, Dr.**
**Amselstrasse 11**
**CH-4123 Alschwil(CH)**

(72) Erfinder: **Kristiansen, Odd, Dr.**
**Delligrabenstrasse 7**
**CH-4313 Möhlin(CH)**

(54) **Substituierte Thioharnstoffe, Isothioharnstoffe und Carbodiimide.**

(57) Neuartige substituierte N-Phenylthioharnstoffe, N-Phenylisothioharnstoffe und N-Phenylcarbodiimide der Formel

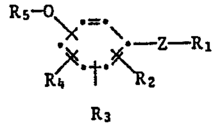

worin

$R_1$ $C_1$-$C_{12}$-Alkyl, $C_3$-$C_8$-Cycloalkyl, polycyclisches Alkyl mit insgesamt 7 bis 10 C-Atomen, mit 1 bis 12 Halogenatomen substituiertes $C_1$-$C_{12}$-Alkyl, mit 1 oder 2 $C_3$-$C_6$-Cycloalkylresten substituiertes $C_1$-$C_4$-Alkyl Alkoxyalkyl mit insgesamt 3 bis 10 C-Atomen, mit einem Phenylrest substituiertes $C_1$-$C_5$-Alkyl, mit einem ein- oder zweifach mit Halogen, Methyl, Methoxy oder Aethoxy substituierten Phenylrest substituiertes $C_1$-$C_5$-Alkyl, $C_3$-$C_{10}$-Alkenyl oder $C_3$-$C_{10}$-Alkinyl;

$R_2$ Wasserstoff oder $C_1$-$C_4$-Alkyl;
$R_3$ $C_1$-$C_4$-Alkyl;
$R_4$ Wasserstoff oder $C_1$-$C_4$-Alkyl; und
$R_5$ einen der Reste

EP 0 256 985 A2

./...

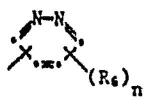 , 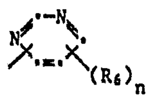 , 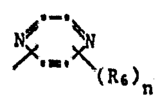 ,

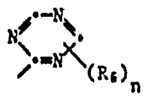 , 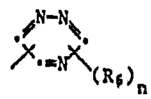 , 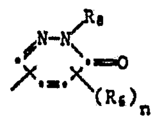 ,

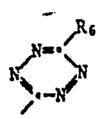 , 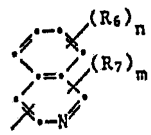 , 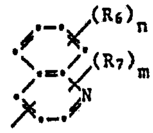 ,

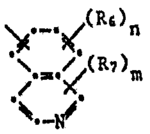 , 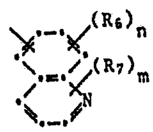 , 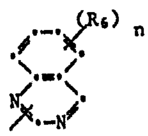 , 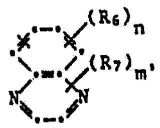 ,

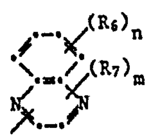 , 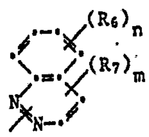 oder 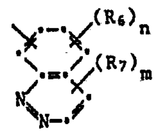 ;

$R_6$ und $R_7$ unabhängig voneinander Halogen, $C_1$-$C_4$-Alkyl, mit 1 bis 7 Halogenatomen substituiertes $C_1$-$C_3$-Alkyl, $C_1$-$C_4$-Alkoxy, mit 1 bis 7 Halogenatomen substituiertes $C_1$-$C_3$-Alkoxy oder Phenyl;

$R_8$ $C_1$-$C_4$-Alkyl;

$R_9$ $C_1$-$C_4$-Alkyl oder $C_3$-$C_4$-Alkenyl;

n eine ganze Zahl von 0 bis 3;

m eine ganze Zahl von 0 bis 2; und

Z eine der Gruppen

```
      S              SR9
      ||              |
–NH–C–NH–, –N=C–NH– oder –N=C=N–
```

bedeuten, sowie deren Salze. Verfahren zur Herstellung dieser Verbindungen sowie diese enthaltende Mittel zur Verwendung in der Schädlingsbekämpfung, speziell zur Bekämpfung von Pflanzen und Tieren befallenden Insekten und Vertretern der Ordung Akarina, insbesondere von pflanzenschädigenden saugenden und fressenden Schädlingen.

CIBA-GEIGY AG
Basel (Schweiz)

5-16051/+

## Substituierte Thioharnstoffe, Isothioharnstoffe und Carbodiimide

Die vorliegende Erfindung betrifft neuartige substituierte N-Phenylthioharnstoffe, N-Phenylisothioharnstoffe und N-Phenylcarbodiimide, Verfahren zu ihrer Herstellung und ihre Verwendung in der Schädlingsbekämpfung.

Diese erfindungsgemässen Verbindungen haben die Formel I

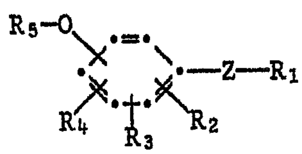

(I),

worin

$R_1$ $C_1$-$C_{12}$-Alkyl, $C_3$-$C_8$-Cycloalkyl, polycyclisches Alkyl mit insgesamt 7 bis 10 C-Atomen, mit 1 bis 12 Halogenatomen substituiertes $C_1$-$C_{12}$-Alkyl, mit 1 oder 2 $C_3$-$C_6$-Cycloalkylresten substituiertes $C_1$-$C_4$-Alkyl, Alkoxyalkyl mit insgesamt 3 bis 10 C-Atomen, mit einem Phenylrest substituiertes $C_1$-$C_5$-Alkyl, mit einem ein- oder zweifach mit Halogen, Methyl, Methoxy oder Aethoxy substituierten Phenylrest substituiertes $C_1$-$C_5$-Alkyl, $C_3$-$C_{10}$-Alkenyl oder $C_3$-$C_{10}$-Alkinyl;

$R_2$ Wasserstoff oder $C_1$-$C_4$-Alkyl;

$R_3$ $C_1$-$C_4$-Alkyl;

$R_4$ Wasserstoff oder $C_1$-$C_4$-Alkyl; und

$R_5$ einen der Reste

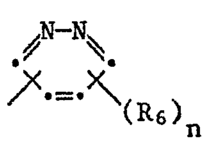 , 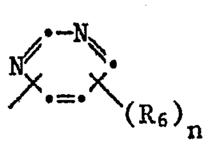 , 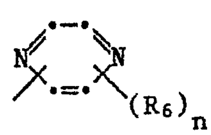 ,

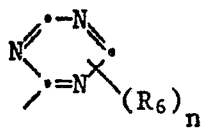 , 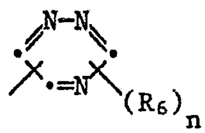 , 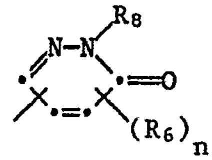 ,

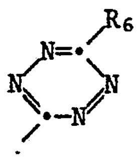 , 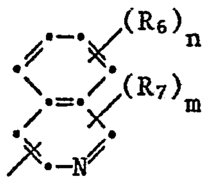 , 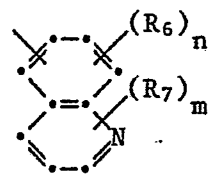 ,

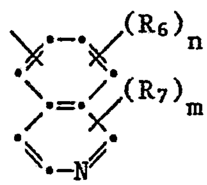 , 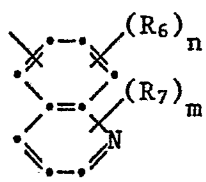 , 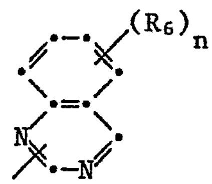 , 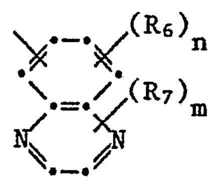 ,

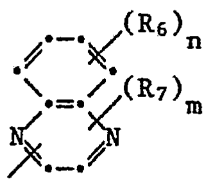 , 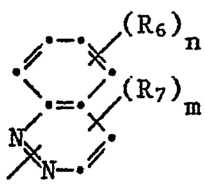 oder 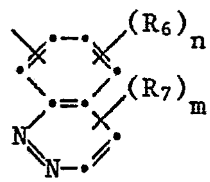 ;

$R_6$ und $R_7$ unabhängig voneinander Halogen, $C_1$-$C_4$-Alkyl, mit 1 bis 7 Halogenatomen substituiertes $C_1$-$C_3$-Alkyl, $C_1$-$C_4$-Alkoxy, mit 1 bis 7 Halogenatomen substituiertes $C_1$-$C_3$-Alkoxy oder Phenyl;

$R_8$ $C_1$-$C_4$-Alkyl;

$R_9$ $C_1$-$C_4$-Alkyl oder $C_3$-$C_4$-Alkenyl;

n eine ganze Zahl von 0 bis 3;

m eine ganze Zahl von 0 bis 2; und

Z eine der Gruppen $-NH-\overset{\overset{S}{\|}}{C}-NH-$, $-N=\overset{\overset{SR_9}{|}}{C}-NH-$ oder $-N=C=N-$

bedeuten, sowie deren Salze.

Die bei $R_1$ bis $R_4$ sowie $R_9$ in Frage kommenden, gegebenenfalls substituierten Alkylgruppen und -Substituenten können geradkettig oder verzweigt sein. Beispiele solcher Gruppen sind dementsprechend z.B. Methyl, Aethyl, Propyl, Isopropyl, n-, i-, sek.-, tert.-Butyl, n-Pentyl, n-Hexyl, n-Heptyl, n-Octyl, n-Nonyl, n-Decyl und deren Isomere.

0256985



Unter Halogen im Rahmen der vorliegenden Erfindung ist vorzugsweise F, Cl und Br zu verstehen, insbesondere F und Cl.

Wegen ihrer Wirkung als Schädlingsbekämpfungsmittel bevorzugt sind Verbindungen der Formel Ia,

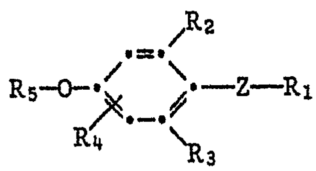 (Ia),

wobei die Reste $R_1$ bis $R_5$ und Z die in Anspruch 1 angegebene Bedeutung haben.

Wertvoll sind aufgrund ihrer biologischen Wirksamkeit ferner solche Verbindungen der Formel I, worin
$R_4$ Wasserstoff bedeutet und solche, worin
Z die Gruppe $-NH-\overset{S}{\overset{\|}{C}}-NH-$ darstellt.

Wegen ihrer guten pestiziden Wirkung herauszustellen sind speziell diejenigen Verbindungen der Formel I, worin Z die Gruppe $-N=\overset{SR_9}{\overset{|}{C}}-NH-$ oder $-N=C=N-$ bedeutet, d.h. die Isothioharnstoffe und Carbodiimide.

Hervorzuheben sind insbesondere Verbindungen der Formel I, die dadurch gekennzeichnet sind, dass $R_5$ einen der Reste

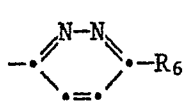 , 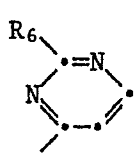 , 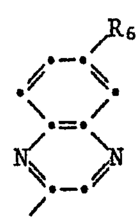 oder 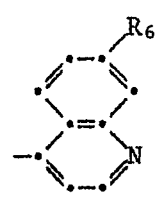

bedeutet, wobei $R_6$ für Halogen oder Trifluormethyl steht.

Weiterhin bevorzugt sind solche Verbindungen der Formel I, worin $R_1$ $C_3$-$C_5$-Alkyl, $R_2$ und $R_3$ unabhängig voneinander $C_1$-$C_3$-Alkyl und $R_5$ Methyl oder Aethyl bedeuten; und auch solche Verbindungen der Formel I worin $R_1$ Isopropyl oder tert.-Butyl, $R_2$ und $R_3$ Methyl oder Aethyl, $R_4$ Wasserstoff und $R_5$ Methyl bedeuten.

Die Verbindungen der Formel I können in Form von Additionssalzen anorganischer oder organischer Säuren vorliegen und erfindungsgemäss auch in Form ihrer Salze verwendet werden. Unter den Verbindungen der Formel I sind im Sinne der der vorliegenden Erfindung demzufolge sowohl die freien Verbindungen der Formel I, bzw. Ia, als auch deren Säureadditionssalze zu verstehen.

Die Verbindungen der Formel I können nach an sich bekannten Verfahren in ihre Säuresalze überführt werden. Zur Bildung von Additionssalzen sind beipielsweise Chlorwasserstoffsäure, Bromwasserstoffsäure, Jodwasserstoffsäure, Salpetersäure, Phosphorsäure, Schwefelsäure, Essigsäure, Propionsäure, Buttersäure, Valeriansäure, Oxalsäure, Malonsäure, Bernsteinsäure, Aepfelsäure, Maleinsäure, Fumarsäure, Milchsäure, Weinsäure, Zitronensäure, Benzoesäure, Phthalsäure, Zimtsäure, Phenylsulfonsäure und Salizylsäure geeignet.

Die Verbindungen der Formel I können nach an sich bekannten Verfahren hergestellt werden (vgl. z.B. die europäischen Patentanmeldungen 0 175 649, 0 008 435 und DE-OS 2 730 620):

A.) Man kann einen Thioharnstoff der Formel I, worin Z die Gruppe $-NH-\overset{S}{\overset{\|}{C}}-NH-$ bedeutet, z.B. dadurch erhalten, dass man eine Verbindung der Formel II

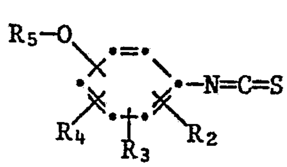

(II)

mit einer Verbindung der Formel III

$R_1$–$NH_2$ (III)

umsetzt, wobei die Reste $R_1$ bis $R_6$ und n die unter Anspruch 1 angegebenen Bedeutungen haben.

Das vorerwähnte Verfahren kann üblicherweise unter normalem Druck und in Gegenwart eines organischen Lösungs-oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; sowie Ketone, z.B. Aceton, Methyläthylketon, Methylisopropylketon und Methylisobutylketon. Das Verfahren wird im allgemeinen bei einer Temperatur von 0 bis 150°C, insbesondere zwischen 10 und 70°C, durchgeführt, vorzugsweise bei Raumtemperatur.

Zu den Isothiocyanaten der Formel II kann man durch Umsetzung der entsprechenden Aniline der Formel V

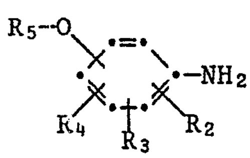

(V)

mit Thiophosgen gelangen. Diese Aniline der Formel V sind z.B. durch Verätherung wie folgt erhältlich:

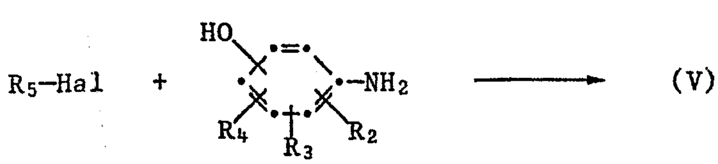

(VI) (VII)

oder

$R_5-SO_2CH_3$ + 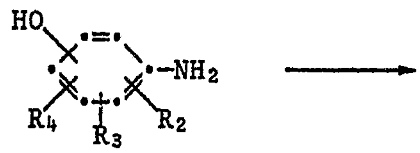 (V) ,

(VIII) (VII)

wobei in den obigen Formeln V, VI, VII und VIII die Reste $R_2$ bis $R_5$ die vorstehend angegebenen Bedeutungen haben. Die Verbindungen der Formeln VI und VIII sowie die Hydroxyaniline der Formel VII sind bekannt und können nach üblichen Verfahren hergestellt werden. Die verätherten Aniline der Formel V und die Isothiocyanate der Formel II sind neue Verbindungen und bilden ebenfalls einen Gegenstand der vorliegenden Erfindung.

B.) Einen Isothioharnstoff der Formel I, worin Z die Gruppe $-N=C(SR_9)-NH-$ bedeutet, kann man dadurch erhalten, dass man eine Thioharnstoffverbindung der Formel I, worin Z die Gruppe $-NH-C(=S)-NH-$ bedeutet, mit einer Verbindung der Formel IV

$R_9-X$ (IV)

umsetzt, wobei $R_9$ die angegebene Bedeutunge gemäss Formel I hat und X eine Abgangsgruppe, z.B. Halogen, wie Chlor, oder Sulfat, oder Methylsulfonat, darstellt. Aus dem Isothiuroniumsalz kann der freie Isothioharnstoff durch Behandlung mit einer Base, z.B. wässriger Alkalilauge, freigesetzt werden.

Das obige Verfahren wird zweckmässig bei einer Temperatur zwischen 10 und 250°C, vorzugsweise 70-200°C, bie normalem oder leicht erhöhtem Druck und vorzugsweise in Gegenwart eines gegenüber den Reaktionsteilnehmern inerten Lösungs- oder Verdünnungsmittels durchgeführt. Als Lösungs- oder Verdünnungsmittel eignen sich z.B.

Aether und ätherartige Verbindungen, wie Diäthyläther, Diisopropyläther, Dioxan und Tetrahydrofuran; aromatische Kohlenwasserstoffe, wie Benzol, Toluol, Xylole; Ketone, wie Aceton, Methyläthylketon und Cyclohexanon; Alkohole und Dimethylformamid. Bevorzugt wird das Verfahren bei Rückflusstemperatur des Reaktionsgemisches vorgenommen.

Die erfindungsgemässen Isothioharnstoffe können in ihren tautomeren Formen

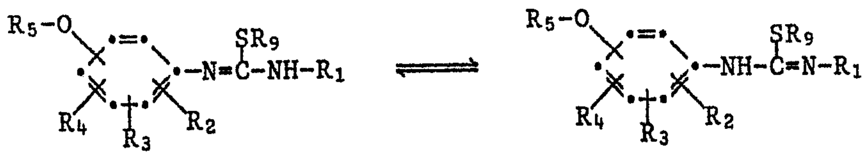

vorliegen. Die Erfindung umfasst beide Formen als solche, sowie auch Tautomerengemische.

C.) Eine Verbindung der Formel I, worin Z die Gruppe $-N=C=N-$ bedeutet, d.h. ein Carbodiimid, kann mann herstellen indem man aus einer Verbindung der Formel I, worin Z die Gruppe $-NH-\overset{S}{\overset{\|}{C}}-NH-$ bedeutet, Schwefelwasserstoff abspaltet. Derartige Abspaltungs-Reaktionen können nach literaturbekannten Arbeitsweisen z.B. mit HgO, bestimmten Pyridiniumsalzen, Chloressigsäureestern, Cyanursäurechlorid, p-Toluolsulfochlorid oder bestimmten Phosphorsäureester-Derivaten durchgeführt werden [T. Shibanuma, Chemistry Letters (1977), p. 575-6; S. Kim, Tetrahedron Letters (1985), p. 1661-1664; W. Weith, B.6 (1873) 1398; G. Amiard, Bull. Soc. chim. 1956, 1360].

Das vorerwähnte Verfahren kann üblicherweise unter normalem Druck und in Gegenwart eines vorzugsweise aprotischen organischen Lösungs- oder Verdünnungsmittels durchgeführt werden. Als Lösungs- oder Verdünnungsmittel eignen sich z.B. Aether und ätherartige Verbindungen, wie Diäthyläther, Dipropyläther, Dibutyläther, Dioxan, Dimethoxyäthan und Tetrahydrofuran; N,N-dialkylierte Carbonsäureamide; aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Nitrile, wie Acetonitril oder Propionitril; sowie Ketone, z.B. Aceton, Methyläthylketon, Methylisopropylketon und Methylisobutylketon. Das Verfahren wird im allgemeinen bei einer Temperatur von 0 bis +150°C, insbesondere zwischen 10 und 50°C, z.B. bei Raumtemperatur, durchgeführt.

Man kann die unter Formel I fallenden Carbodiimide auch in an sich bekannter Weise herstellen durch Umsetzung von entsprechend substituierten Isocyaniddichloriden der Formel IX mit einem Salz des jeweils erwünschten primären Amins der Formel X (vgl. US-PS 3.231.610)

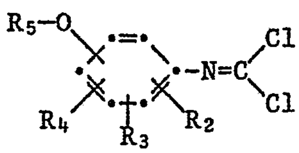 + $R_1NH_2 \cdot HA$ → (I),

(IX) (X)

wobei in den Formeln IX und X die Reste $R_1$ bis $R_5$ die vorstehend unter Formel I angegebenen Bedeutungen haben und A ein Anion, z.B. $Cl^{\ominus}$, bedeutet.

Bei dieser Umsetzung sind als primäre Aminsalze z.B. die Hydrohalogenide geeignet. Die Umsetzung wird vorzugsweise ausgeführt in Gegenwart eines inerten organischen Lösungsmittels von relativ hohem Siedepunkt, wie z.B. chlorierte Benzole, Nitrobenzol, Dimethylacetamid oder Tetramethylensulfon. Als Lösungsmittel können z.B. die folgenden in Betracht kommen: hochsiedende aliphatische, cycloaliphatische und aromatische Kohlenwasserstoffe, wie p-Chlorbrombenzol, 1-Chlornaphthalin oder halogenierte Xylole. Bevorzugt wird im allgemeinen eine Umsetzungstemperatur von 80 bis 200°C.

Die Ausgangsstoffe der Formeln IX und X sind bekannt und können - falls sie neu sind - analog bekannter Arbeitsweisen erhalten werden (vgl. BE-PS 863.078, DE-Patentanmeldung 1.094.737 und US-PS 3.932.507).

Die Verbindungen der Formel I eignen sich insbesondere zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina, insbesondere von pflanzenschädigenden Akarinen, wie z.B. Spinnmilben.

Die gute pestizide Wirkung der erfindungsgemässen Verbindungen der Formel I entspricht einer Abtötungsrate (Mortalität) von mindestens 50-60 % der erwähnten Schädlinge.

Neben ihrer sehr günstigen Wirkung gegenüber Fliegen, wie z.B. Musca domestica und Mückenlarven, eignen sich Verbindungen der Formel I vor allem zur Bekämpfung von pflanzenschädigenden saugenden und fressenden Insekten sowie Akarinen in Zier- und Nutzpflanzungen, insbesondere in Baumwollkulturen (z.B. Spodoptera littoralis und Heliothis virescens) sowie in Gemüsekulturen (z.B. Leptinotarsa decemlineata und Pieris brassicae). Hervorzuheben ist auch die larvizide und ovizide Wirkung von Verbindungen der Formel I. Werden Verbindungen der Formel I von adulten Insekten mit dem Futter aufgenommen, so ist in vielen Fällen, insbesondere bei Coleopteren, wie z.B. Anthonomus grandis, eine verminderte Ei-Ablage und/oder reduzierte Schlupfrate festzustellen.

Die Verbindungen der Formel I sind weiterhin zur Bekämpfung von Ektoparasiten, z.B. von Lucilia sericata, sowie von Zecken an Haus- und Nutztieren geeignet, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt

werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8 bis 22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder

niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag
München/Wien 1981.

Die pestiziden Zubereitungen enthalten in der Regel 0,1 bis 99 %, insbesondere 0,1 bis 95 %, Wirkstoff der Formel I oder Kombinationen dieser Wirkstoffe mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effeke enthalten.

<u>Beispiel 1</u>:

a) <u>Herstellung von 2,6-Diäthyl-4-(6'-chlorpyridazin-3'-yloxy)-anilin:</u>

Es werden 17,3 g 2,6 Diäthyl-4-hydroxyanilin und 23,2 g pulverisiertes Kaliumcarbonat in 80 ml Dimethylsulfoxyd während 30 Minuten bei Raumtemperatur verrührt. Danach wird die Mischung auf 80-90°C erwärmt und eine Lösung von 14,9 g 3,6-Dichlorpyridazin in 20 ml Dimethylsulfoxyd zugetropft. Das Reaktionsgemisch wird 12 Stunden bei 80-90°C nachgerührt. Zur Aufarbeitung wird am Hochvakuum der grösste Teil des Lösungsmittels entfernt und der Rückstand in

Dichlormethan (200 ml) und Wasser (100 ml) aufgenommen. Die organische Phase wird mit Wasser (100 ml) gewaschen, über Natriumsulfat getrocknet und das Lösungsmittel entfernt, wonach man ein braunes Oel erhält. Zur Reinigung wird dieses Rohprodukt in ca. 500 ml eines Gemisches aus Dichlormethan und Essigester (Vol.-Verhältnis 9:1) aufgenommen und über Kieselgel filtriert.

Nach dem Eindampfen der reinen Fraktionen erhält man die Titelverbindung der Formel

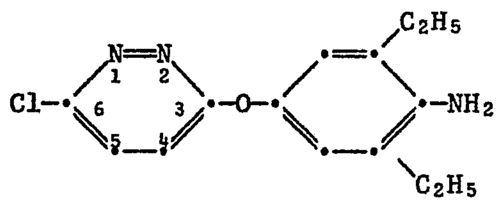

als gelbliches Oel.( Verbindung Nr. 1.1)

In analoger Weise werden die folgenden Aniline der Formel V hergestellt:

| Verbindung Nr. | Formel | Smp. |
|---|---|---|
| 1.2 | 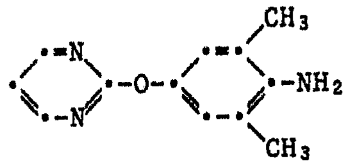 | 199-201°C |
| 1.3 | 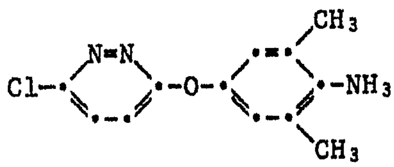 | 161-163°C |
| 1.4 | 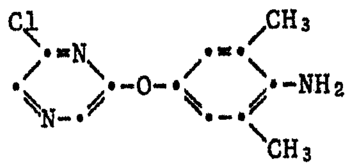 | 155-157°C |
| 1.5 | 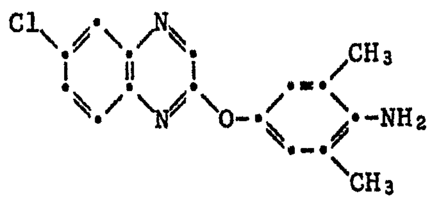 | 153-165.5°C (enthält ca. 10% 7-Cl-Isomeres) |
| 1.6 | 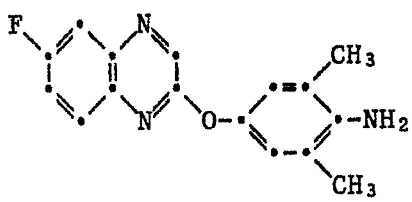 | 166-170°C |
| 1.7 | 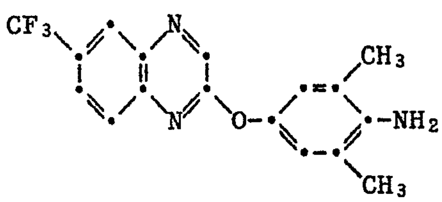 | 140-142°C |
| 1.8 | 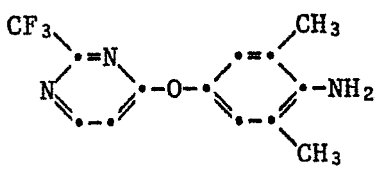 | 142-144°C |

| Verbindung Nr. | Formel | Smp. |
|---|---|---|
| 1.9 | 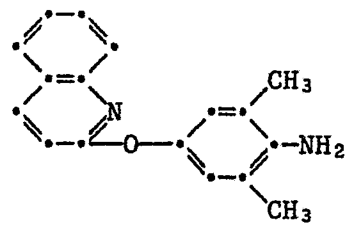 | 182-184°C |
| 1.10 | 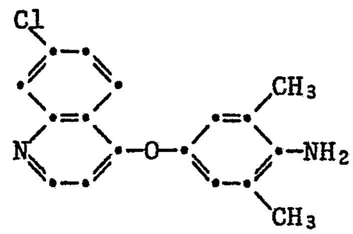 | 159-161°C |
| 1.11 | 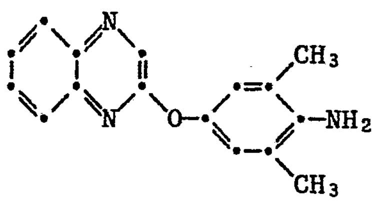 | 198-200°C (Zers.) |
| 1.12 | 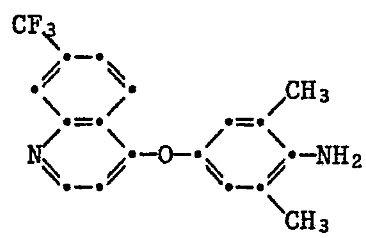 | 110-111°C |

Die folgenden Aniline der Formel V sind ebenfalls wie vorstehend angegeben erhältlich:

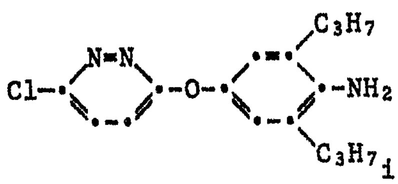

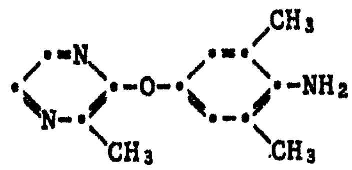

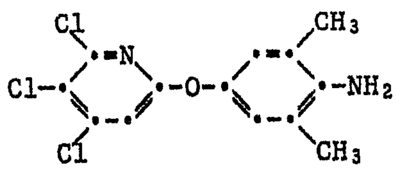

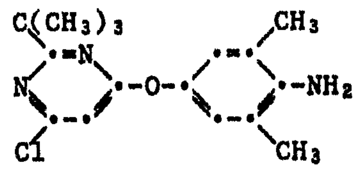

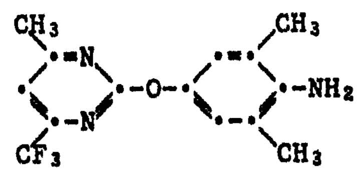

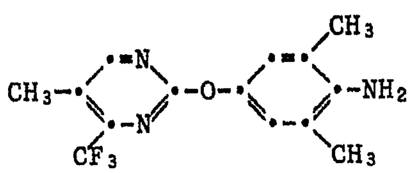

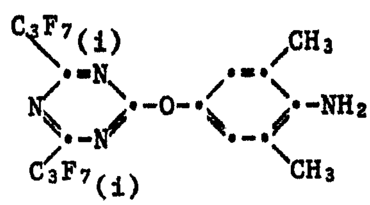

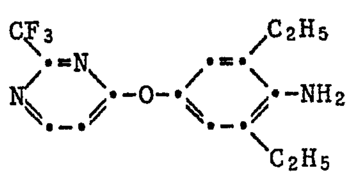

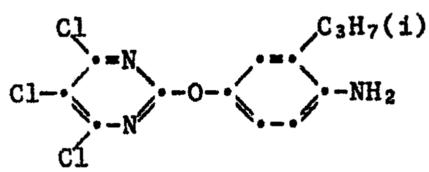

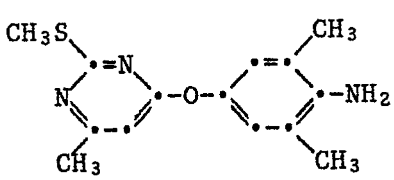

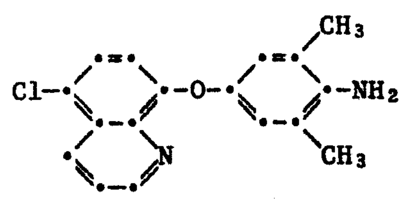

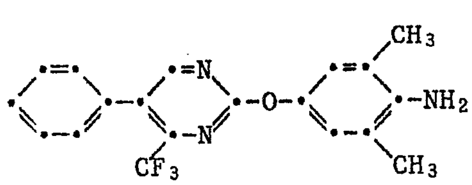

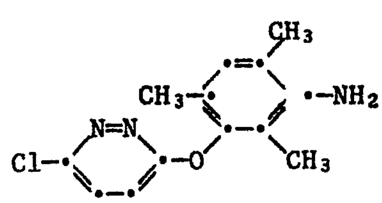

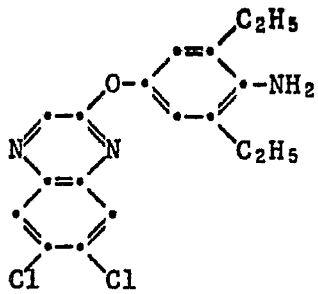

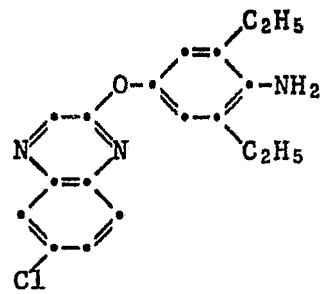

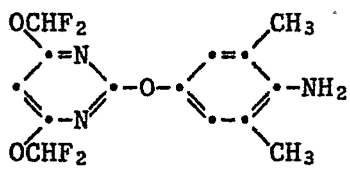

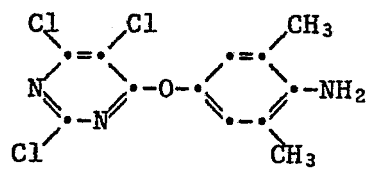

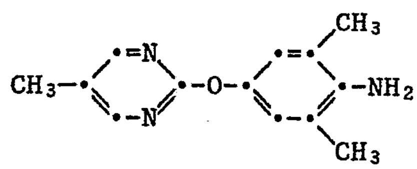

b.) <u>Herstellung von 2,6-Diäthyl-4-(6'-chlorpyridazin-3'-yloxy)-phenylisothiocyanat:</u>

Es werden 25,8 g Thiophosgen und 37,5 g Calciumcarbonat in 300 ml Dichlormethan und 190 ml Wasser verrührt. Bei 0° bis 5°C wird zu diesem Gemisch eine Lösung von 52,0 g 2,6-Diäthyl-4-(6'-chlorpyridazin-3'-yloxy)-anilin in 200 ml Dichlormethan zugetropft.

Das Reaktionsgemisch wird während 5 Stunden bei Raumtemperatur gerührt und danach filtriert. Von dem Filtrat wird die organische Phase abgetrennt, zweimal mit 100 ml Wasser gewaschen, über Natriumsulfat getrocknet und eingedampft.

Der erhaltene feste Rückstand wird in ca. 1000 ml heissem Hexan umkristallisiert.

Man gelangt so zu der Titelverbindung der Formel

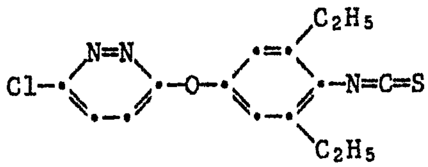

als hellbraunes kristallines Produkt mit einem Schmelzpunkt von 102-104°C (Verbindung Nr. 2.1).

In analoger Weise werden die folgenden Isothiocyanate der Formel II hergestellt:

| Verbindung Nr. | Formel | Smp. |
|---|---|---|
| 2.2 | 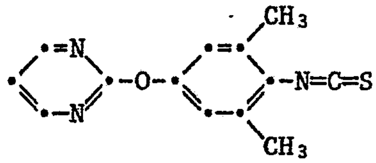 | 102-104°C |
| 2.3 | 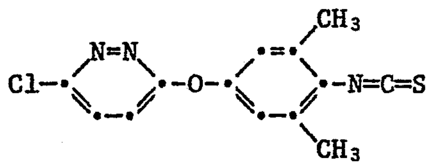 | 129-131°C |
| 2.4 | 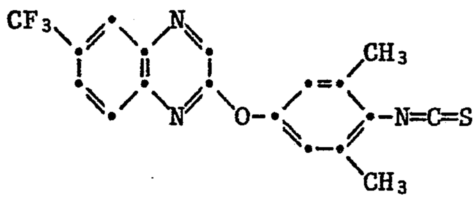 | 118-120°C |
| 2.5 | 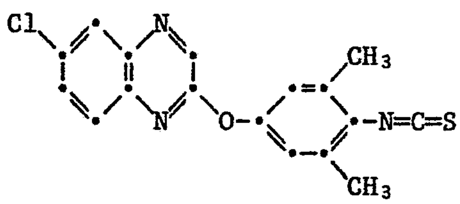 | 126-127°C |
| 2.6 | 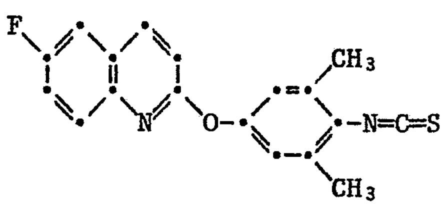 | 128-130°C |
| 2.7 | 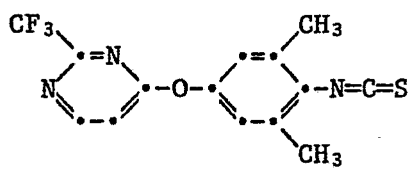 | 158-161°C |

| Verbindung Nr. | Formel | Smp. |
|---|---|---|
| 2.8 | 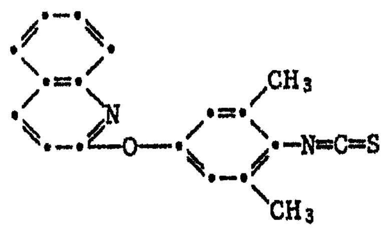 | 145-146°C |
| 2.9 | 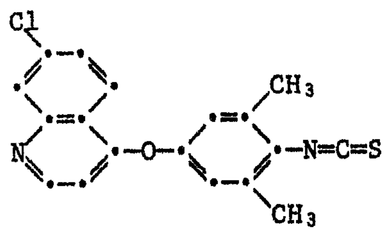 | 110-111°C |
| 2.10 | 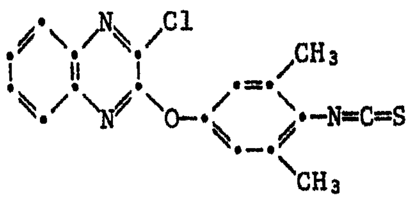 | 135-136°C |
| 2.11 | 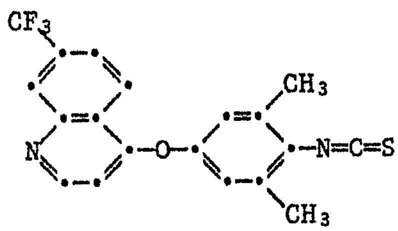 | 105-107°C |

c.) Herstellung von N-2,6-Diäthyl-4-(6'-chlorpyridazin-3'-yloxy)-phenyl-N'-isopropyl-thioharnstoff:

Es werden 23,0 g 2,6-Diäthyl-4-(6'-chlorpyridazin-3'-yloxy)-phenylisothiocyanat zusammen mit 12,8 g Isopropylamin in 100 ml Toluol während 12 Stunden bei Raumtemperatur miteinander verrührt. Die Reaktionsmischung wird dann stark eingeengt, mit Hexan versetzt und zum Auskristallisieren stehen gelassen. Nach dem Abnutschen und Trocknen erhält man die Titelverbindung der Formel

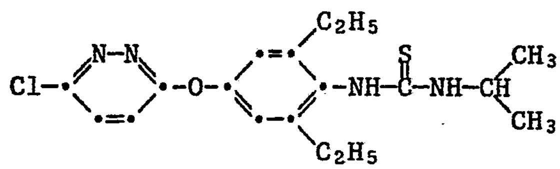

als hellbraunes kristallines Produkt mit einem Schmelzpunkt von 174-6°C (Verbindung Nr. 3.1).

In analoger Weise werden die folgenden Thioharnstoffe der Formel I hergestellt:

| Verbindung Nr. | Formel | Smp. |
|---|---|---|
| 3.2 | 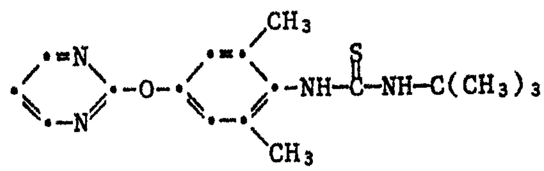 | 142-144°C |
| 3.3 | 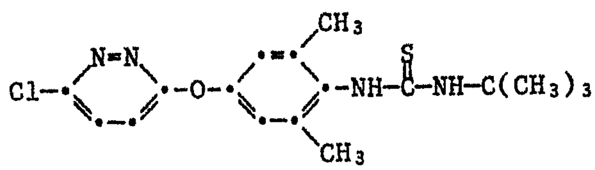 | 188°C |
| 3.4 | 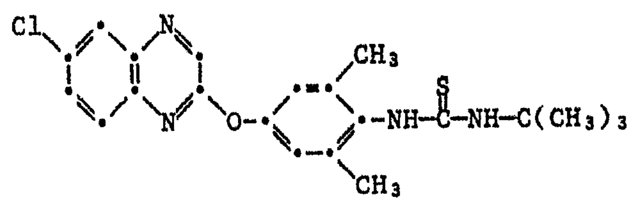 | 140-141,5°C |
| 3.5 | 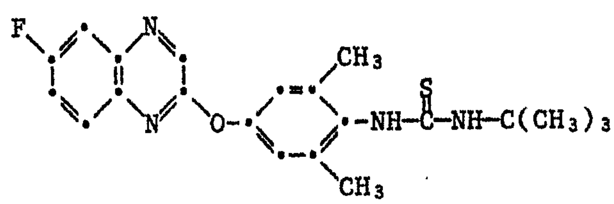 | 148-150°C |
| 3.6 | 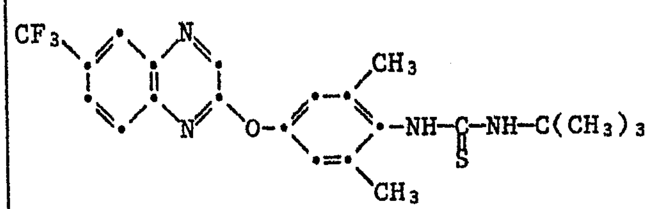 | 148-150°C |
| 3.7 | 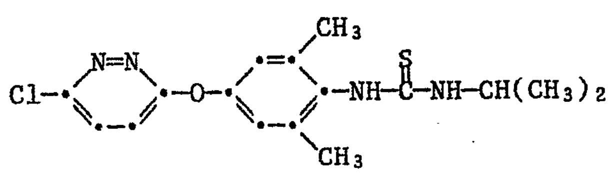 | 184-185°C |
| 3.8 | 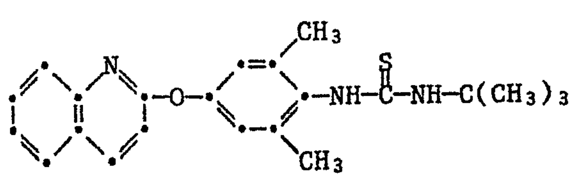 | 143-144°C |
| 3.9 | 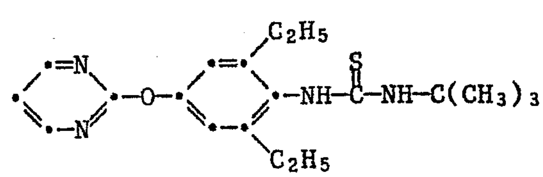 | 153°C (Zers.) |

| Verbindung Nr. | Formel | Smp. |
|---|---|---|
| 3.10 | 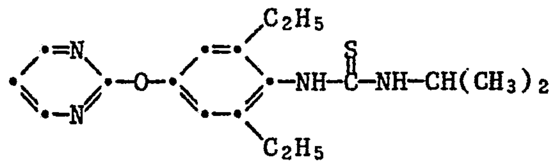 | 120-125°C (Zers.) |
| 3.11 | 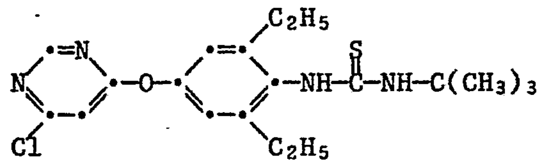 | 139-140°C (Zers.) |
| 3.12 | 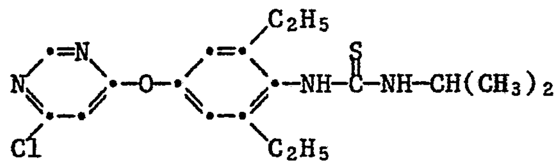 | 165-167°C |
| 3.13 | 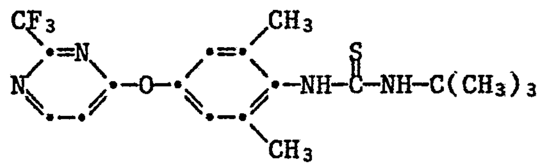 | 142-144°C |
| 3.14 | 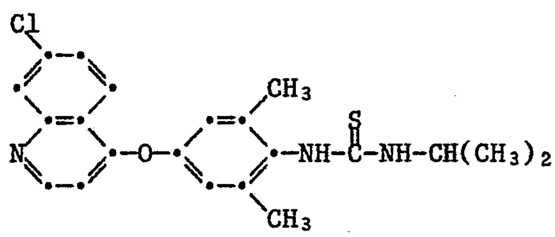 | 170-175,5°C (Zers.) |
| 3.15 | 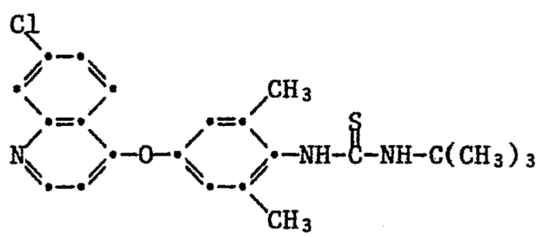 | 158-162°C (Zers.) |
| 3.16 | 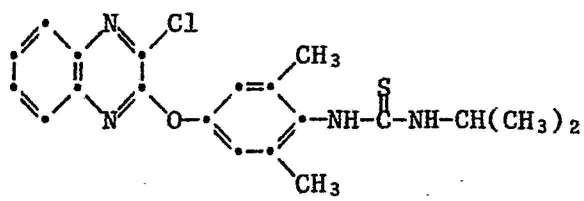 | 200-203°C (Zers.) |

| Verbindung Nr. | Formel | Smp. |
|---|---|---|
| 3.17 | 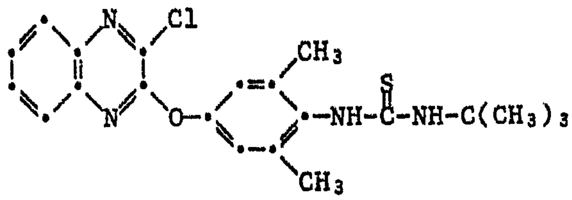 | 175-180°C (Zers.) |
| 3.18 | 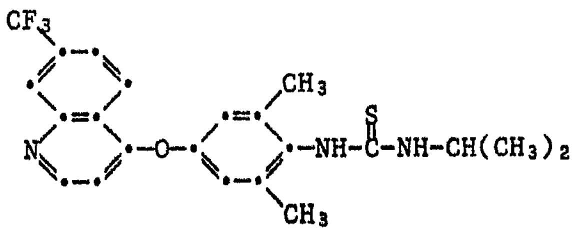 | 181-185°C (Zers.) |
| 3.19 | 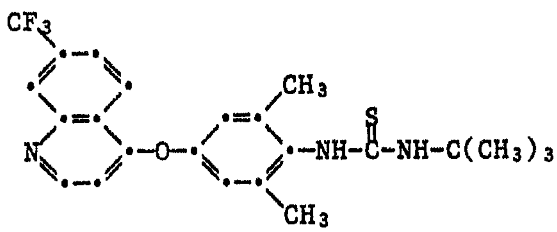 | 168-173°C (Zers.) |

Wie vorstehend angegeben sind auch die folgenden Thioharnstoffe der Formel I herstellbar:

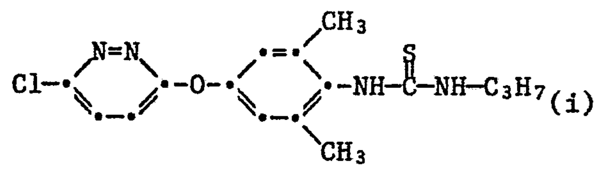

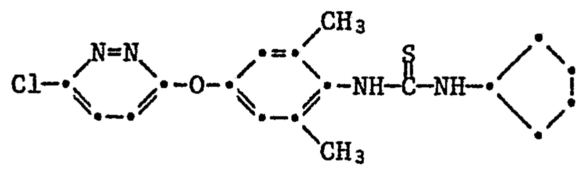

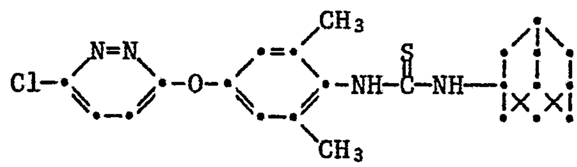

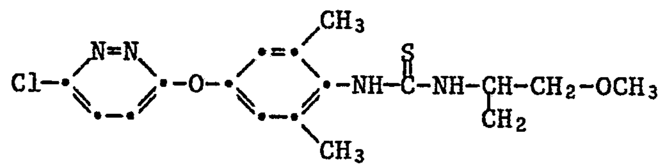

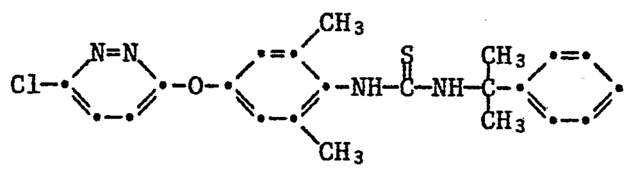

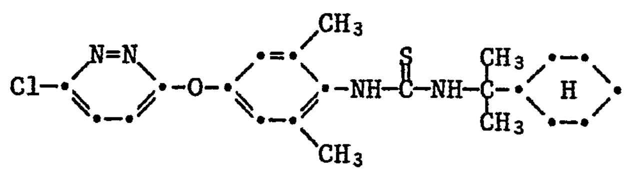

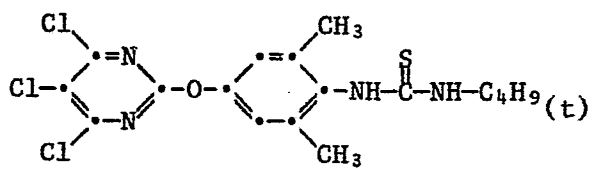

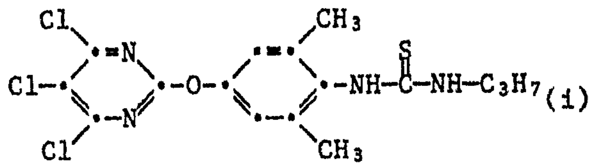

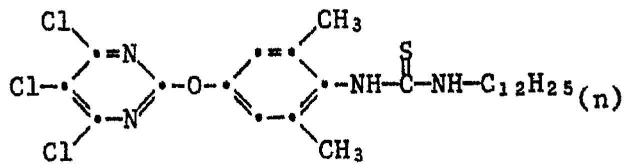

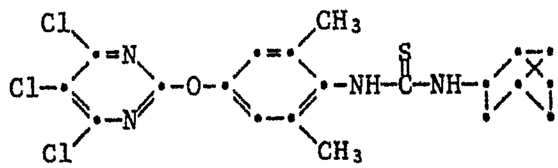

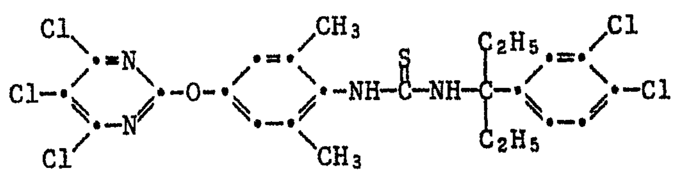

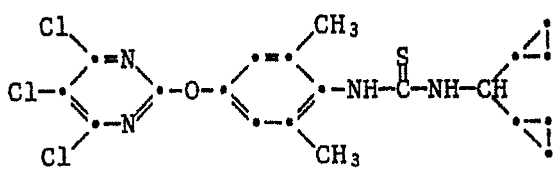

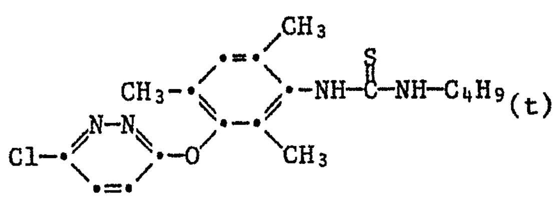

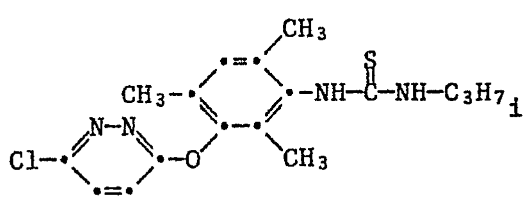

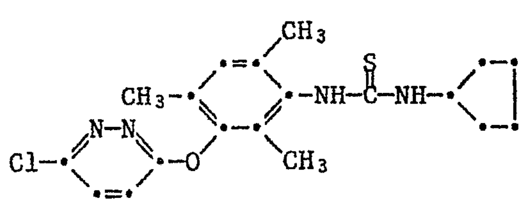

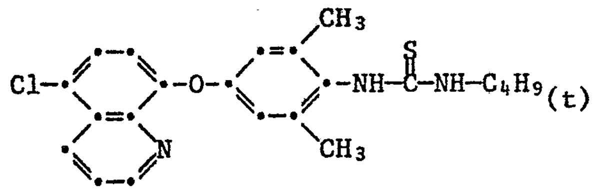

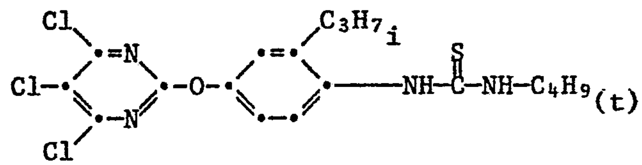

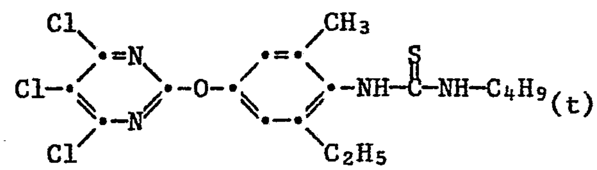

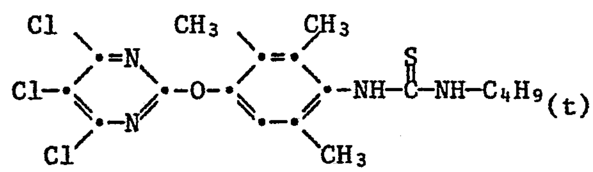

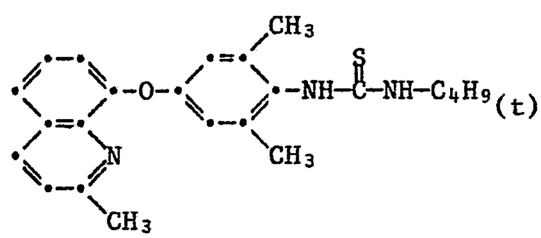

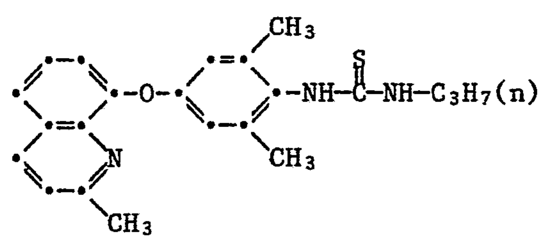

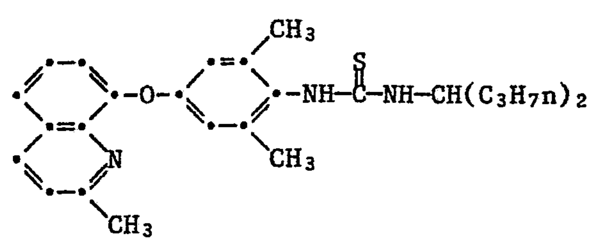

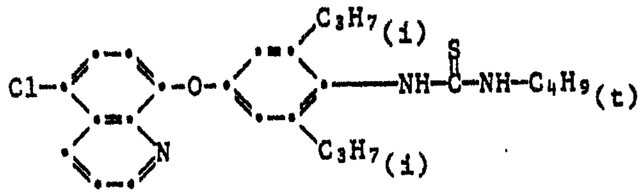

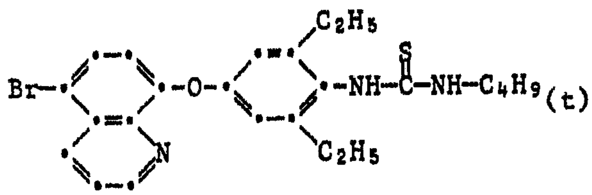

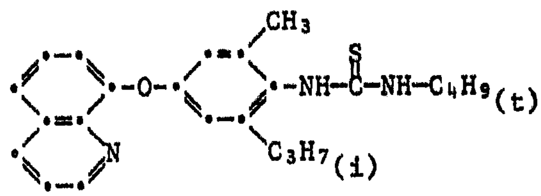

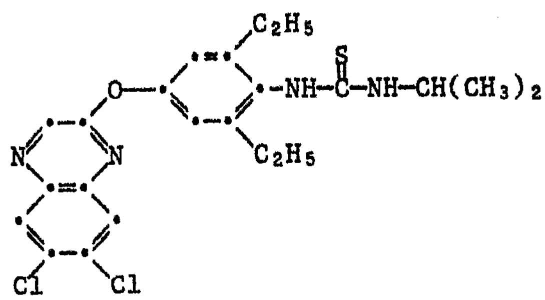

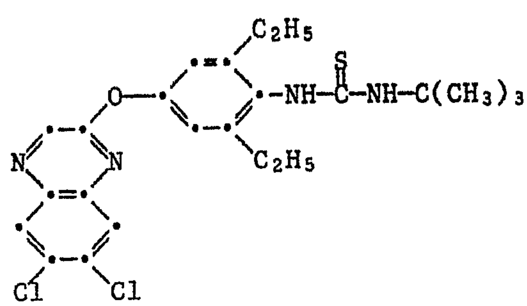

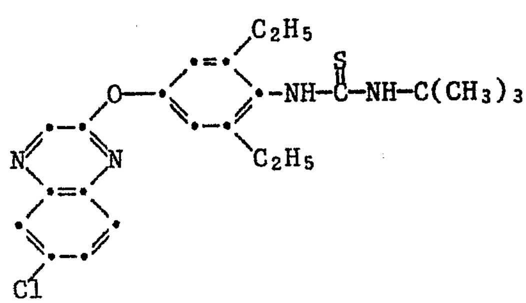

Beispiel 2: Herstellung von N-2,6-Dimethyl-4-(pyrimidin-2'-yloxy)-phenyl-N'-tert.butyl-S-methyl-isothioharnstoff:

Es werden 5,0 g N-2,6-Dimethyl-4-(pyrimidin-2'-yloxy)-phenyl-N'-tert.butyl-thioharnstoff in 40 ml absolutem Aethanol vorgelegt, mit 2,8 g Methyljodid versetzt und während 40 Stunden bei leichtem Rückfluss gerührt. Die erhaltene Reaktionslösung wird eingedampft und der Rückstand in Dichlormethan und 10 %iger wässriger Sodalösung aufgenommen. Die organische Phase wird mit Wasser gewaschen, über Natriumsulfat getrocknet und stark eingeeengt. Diese konzentrierte Lösung wird über Kieselgel filtriert. Es wird mit Dichlormethan nachgewaschen. Nach dem Eindampfen des Filtrates erhält man die Titelverbindung der Formel

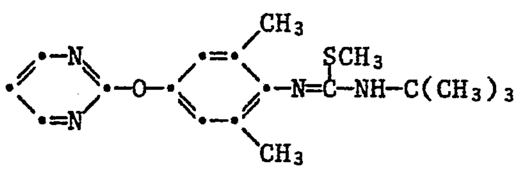

als festes Produkt mit einem Schmelzpunkt von 174-176°C (Verbindung Nr. 4.1).

In analoger Weise werden die folgenden Isothioharnstoffe der Formel I hergestellt:

| Verbindung Nr. | Formel | physikalische Daten |
|---|---|---|
| 4.2 | 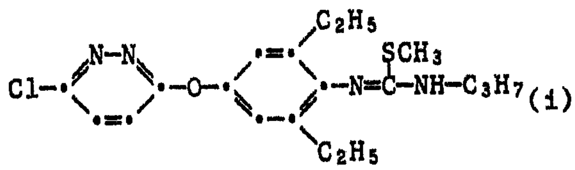 | gelbliches Oel |
| 4.3 | 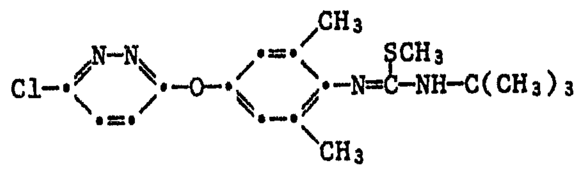 | F=105-107°C |
| 4.4 | 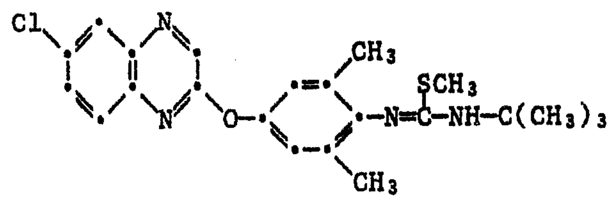 | F=114-116°C |
| 4.5 | 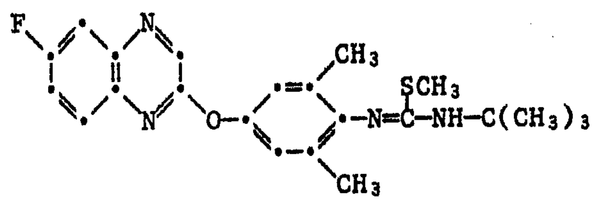 | F=126-128°C |
| 4.6 | 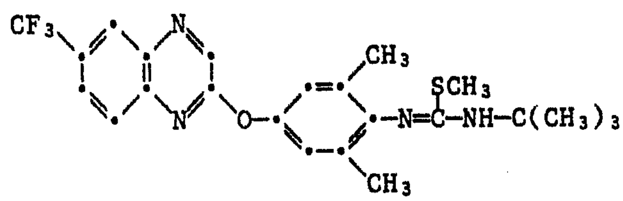 | F=121-123°C |
| 4.7 | 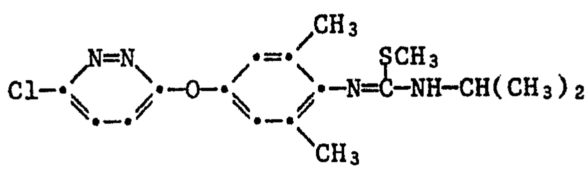 | F=111-113°C |
| 4.8 | 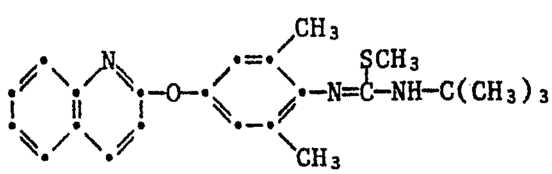 | F=113-115°C |
| 4.9 | 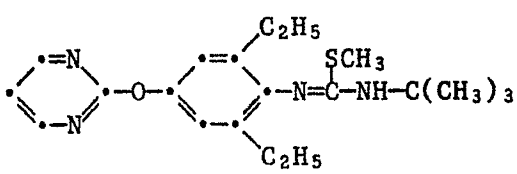 | F=135-136°C |

| Verbindung Nr. | Formel | physikalische Daten |
|---|---|---|
| 4.10 | 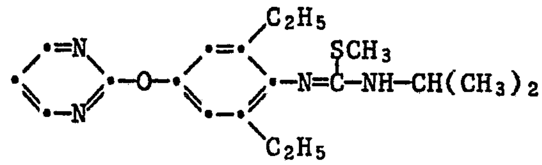 | F=99-101°C |
| 4.11 | 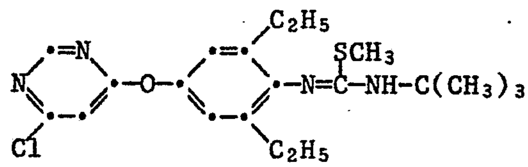 | harzartige Masse |
| 4.12 | 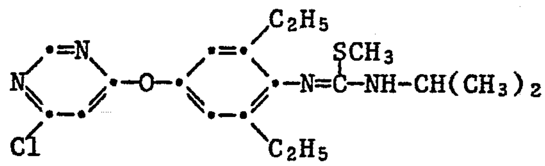 | $n_D^{38}$=1,5774 |
| 4.13 | 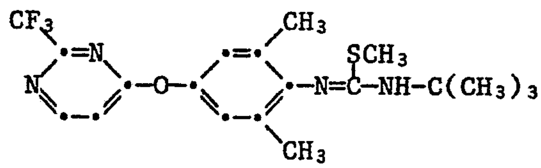 | F=87-89°C |
| 4.14 | 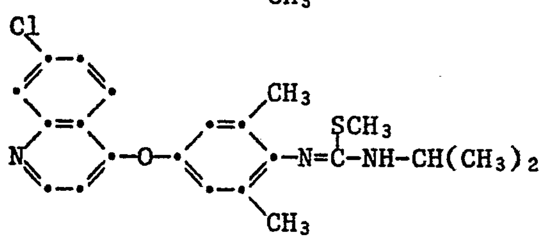 | 46,5-53,5°C |
| 4.15 | 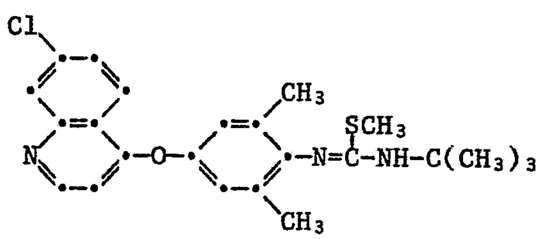 | 117-120°C |
| 4.16 | 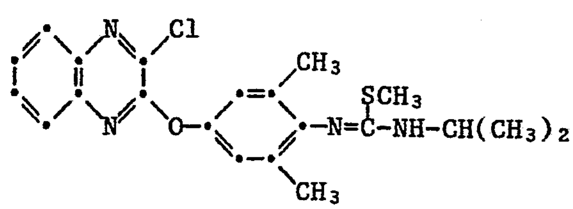 | 120-121,5°C (HJ-Salz: Smp. 171-176°C Zers.) |

| Verbindung Nr. | Formel | Smp. |
|---|---|---|
| 4.17 | 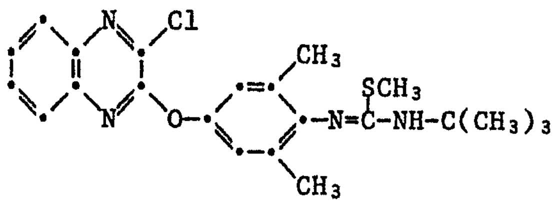 | 136-141,5°C |
| 4.18 | 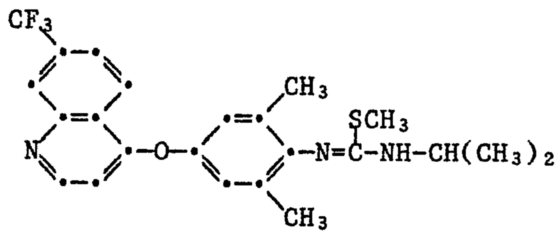 | 45-50°C |
| 4.19 | 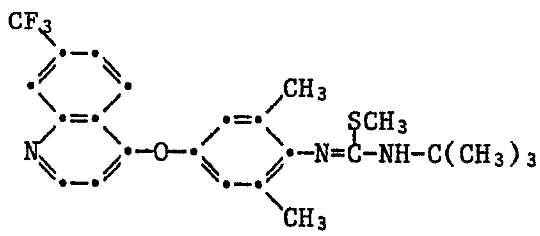 | |

Wie vorstehend angegeben sind auch die folgenden Isothioharnstoffe der Formel I sowie deren Jodwasserstoff-, Oxalsäure- und Phenylsulfonsäure-Salze herstellbar:

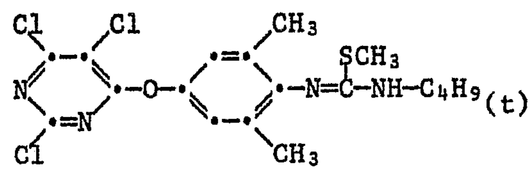

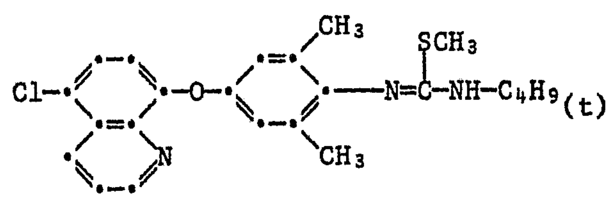

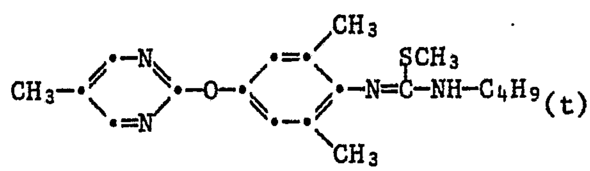

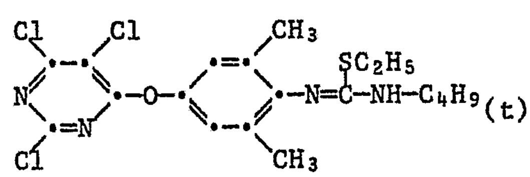

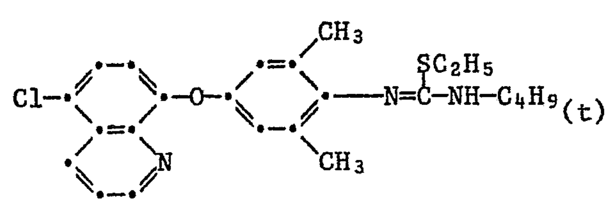

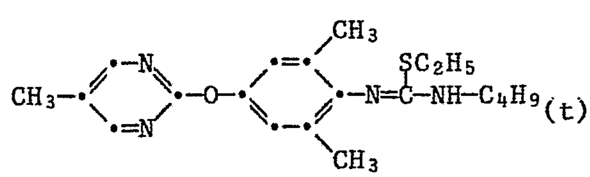

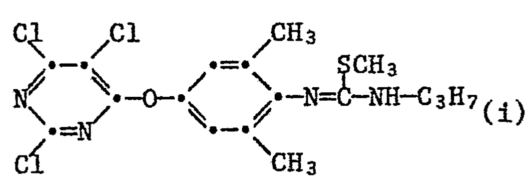

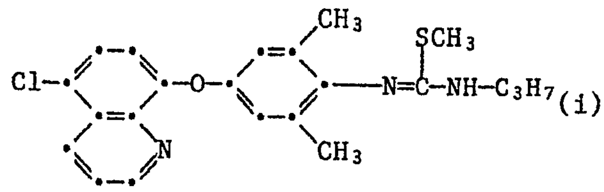

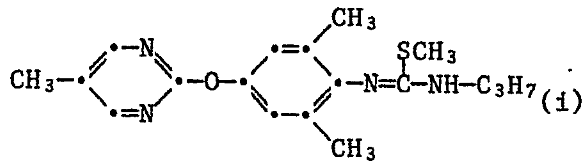

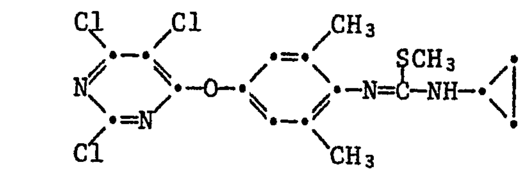

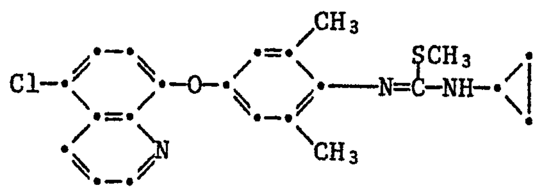

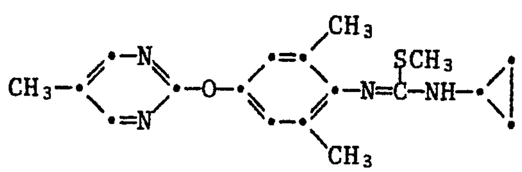

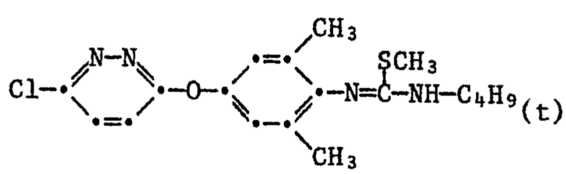

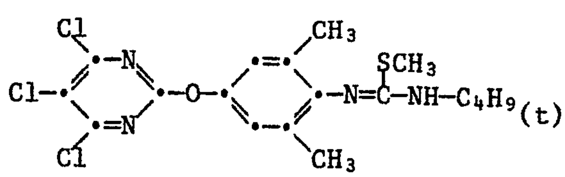

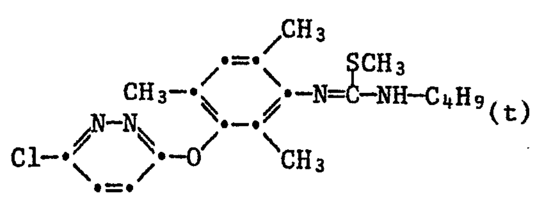

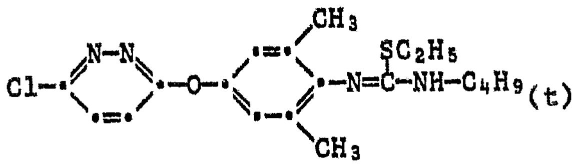

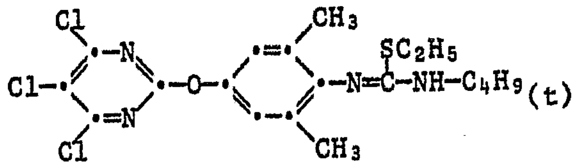

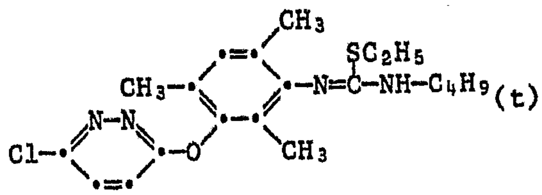

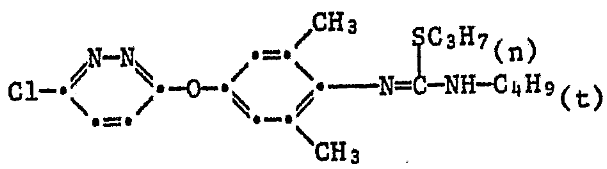

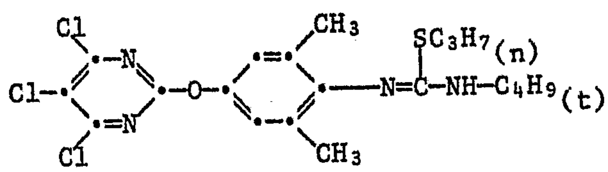

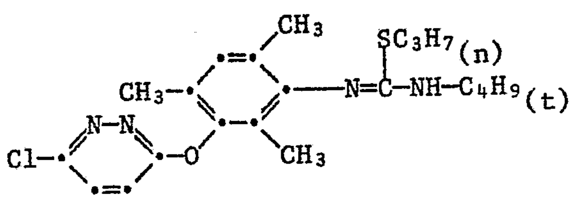

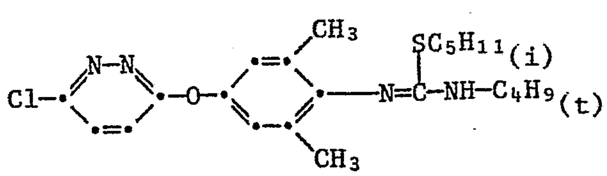

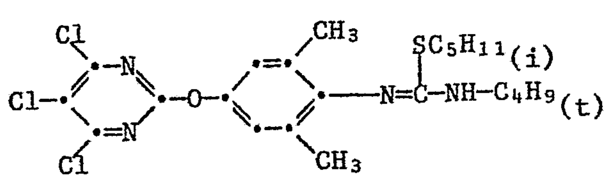

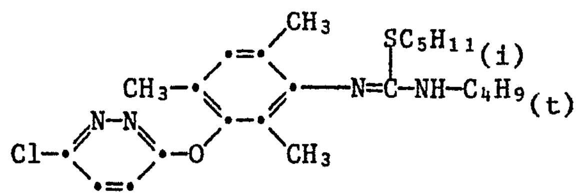

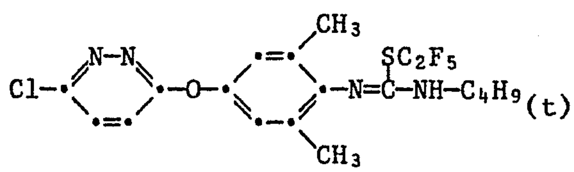

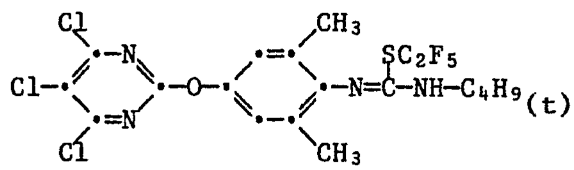

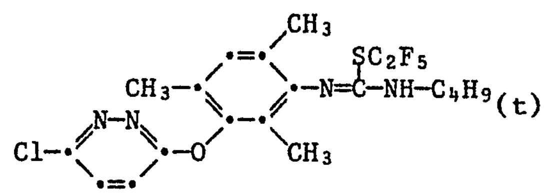

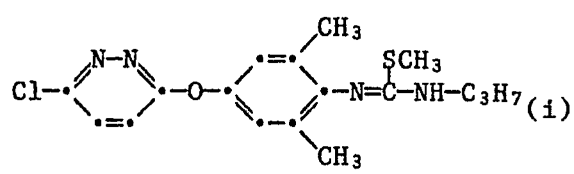

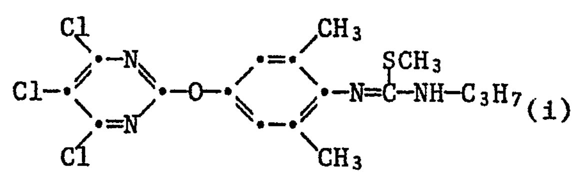

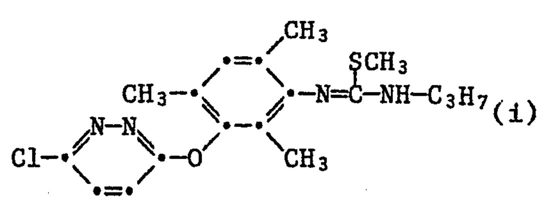

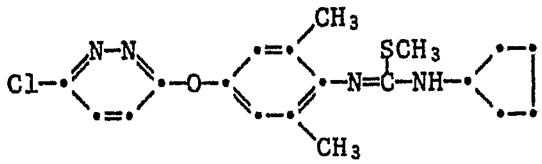

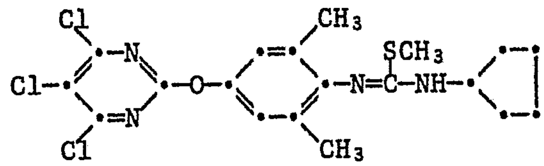

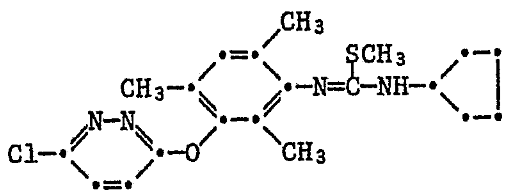

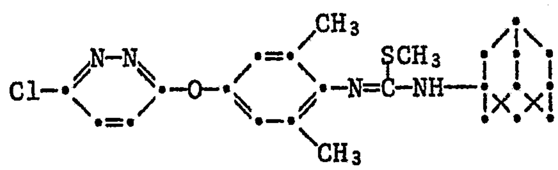

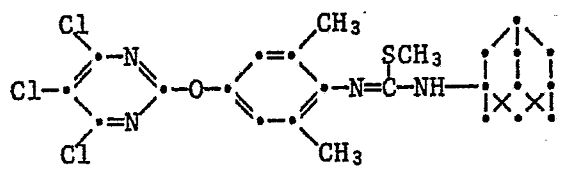

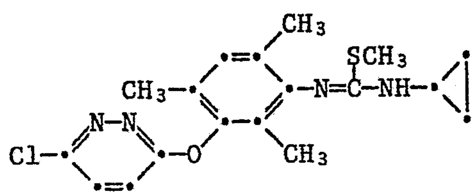

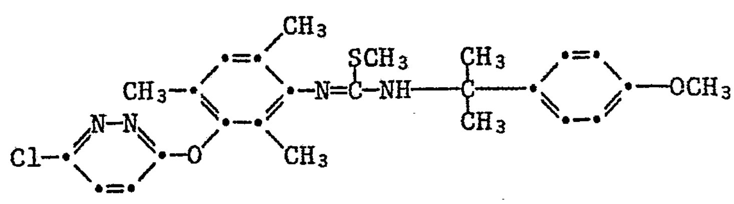

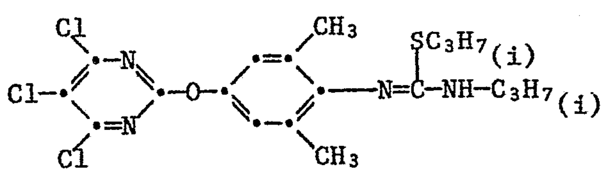

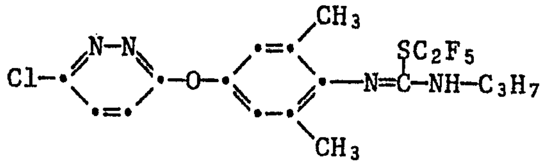 (i)

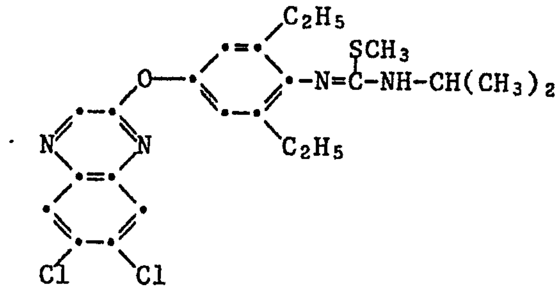

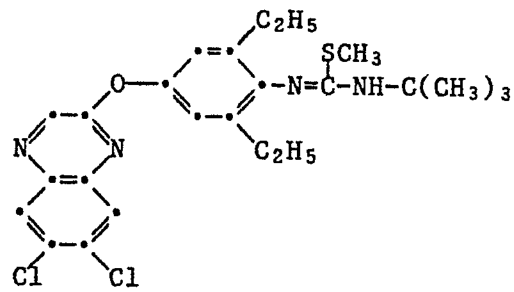

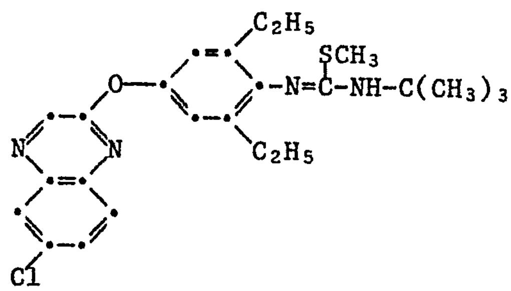

<u>Beispiel 3:</u> <u>Herstellung von N-2,6-Diäthyl-4-(6'-chlorpyridazin-3'-yloxy)-phenyl-N'-isopropyl-carbodiimid:</u>

Es werden 9,85 g N-2,6-Diäthyl-4-(6'-chlorpyridazin-3'-yloxy)-phenyl-N'-isopropyl-thioharnstoff und 6,65 g 2-Chlor-1-methyl-pyridiniumjodid in 50 ml Acetonitril vorgelegt. Bei Raumtemperatur wird unter Rühren eine Lösung von 6,6 g Triäthylamin in 70 ml Acetonitril zugetropft und während 12 Stunden bei leichtem Rückfluss

gerührt. Das Reaktionsgemisch wird dann am Rotationsverdampfer bei 50°C eingedampft. Der Rückstand wird in 1000 ml Hexan aufgenommen, zweimal mit Wasser gewaschen, über Natriumsulfat getrocknet, filtriert und eingedampft.

Man erhält die Titelverbindung der Formel

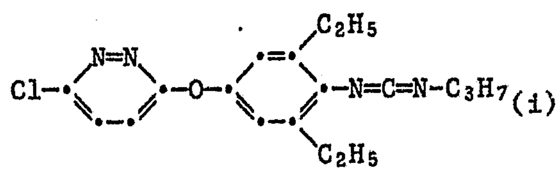

als hellgelbliches Produkt mit einem Schmelzpunkt von 87-89°C (Verbindung Nr. 5.1).

In analoger Weise werden die folgenden Carbodiimide der Formel I hergestellt:

| Verbindung Nr. | Formel | physikalische Daten |
|---|---|---|
| 5.2 | 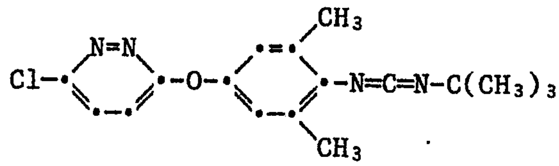 | 110-112°C |
| 5.3 | 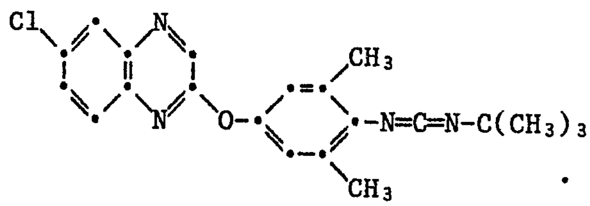 | 100-101.5°C |
| 5.4 | 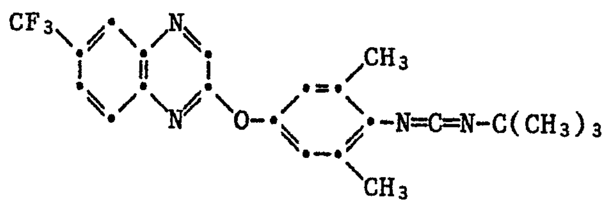 | 107-109°C |
| 5.5 | 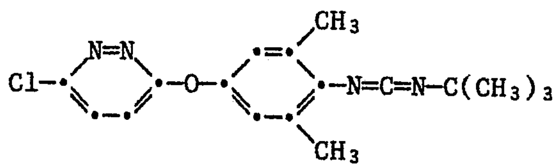 | F=84-86°C |
| 5.6 | 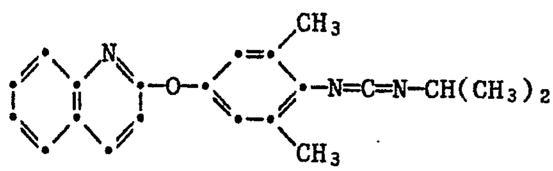 | F=76-79°C |
| 5.7 | 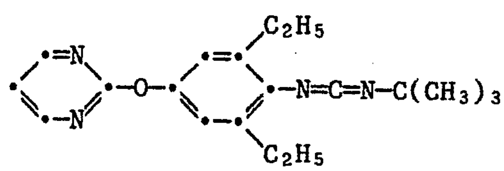 | F=106-107°C |
| 5.8 | 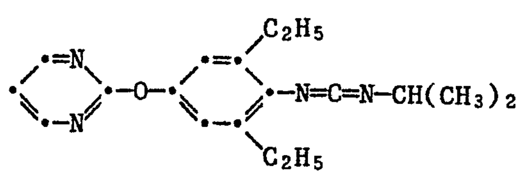 | F=78-80°C |

| Verbindung Nr. | Formel | physikalische Daten |
|---|---|---|
| 5.9 | 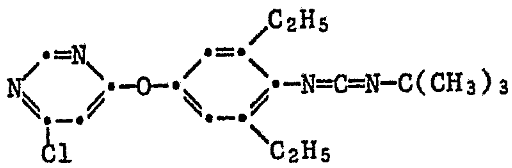 | $n_D^{23}$=1,5688 |
| 5.10 | 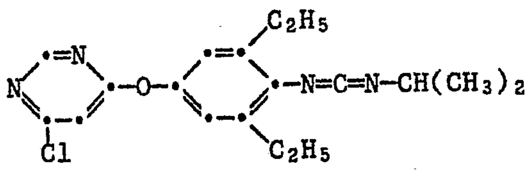 | $n_D^{23}$=1,5775 |
| 5.11 | 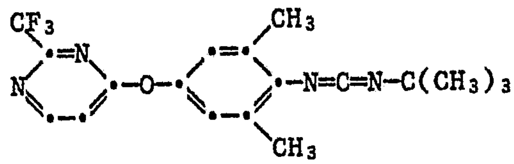 | F=67-69°C |
| 5.12 | 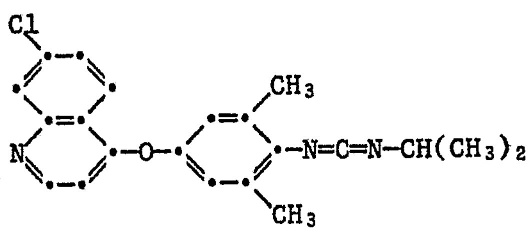 | F=67-69,5°C |
| 5.13 | 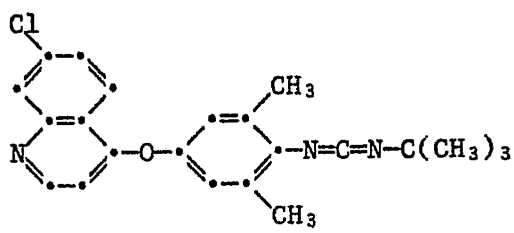 | F=103-107°C |
| 5.14 | 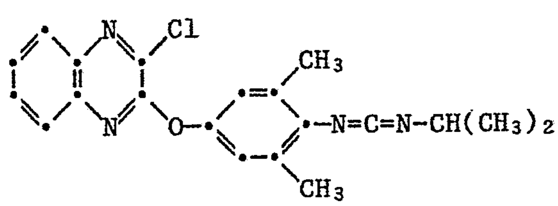 | F=64-67,5°C |
| 5.15 | 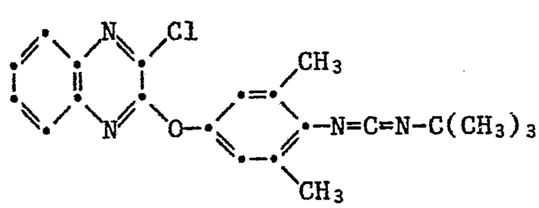 | F=64,5-68°C |

| Verbindung Nr. | Formel | Smp. |
|---|---|---|
| 5.16 | 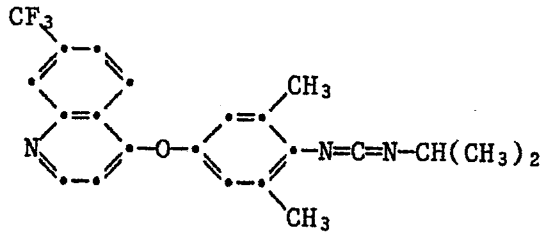 | F=68-70°C |
| 5.17 | 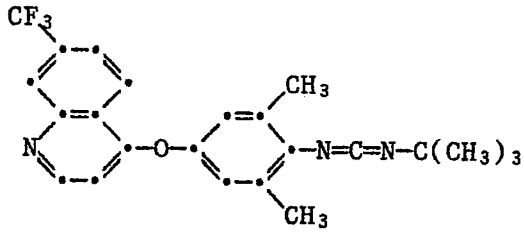 | F=69-71°C |

Wie vorstehend angegeben sind auch die folgenden Carbodiimide der Formel I erhältlich:

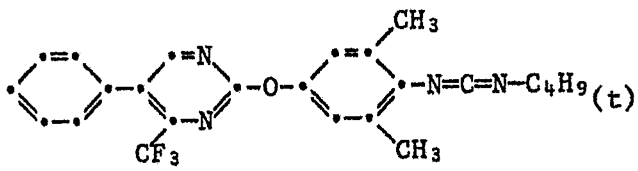

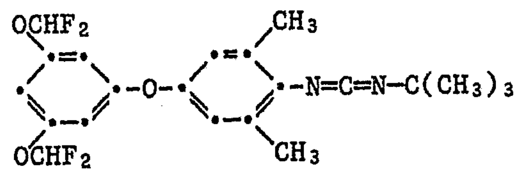

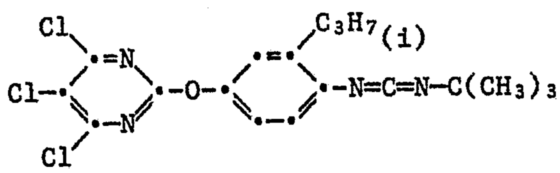

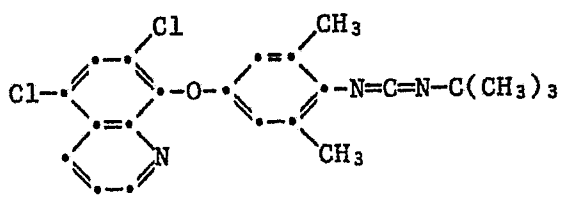

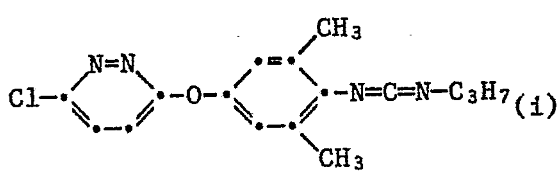

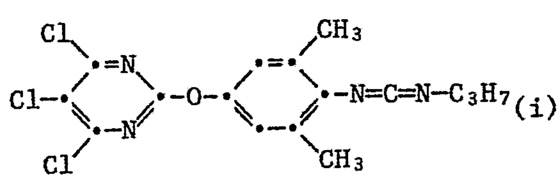

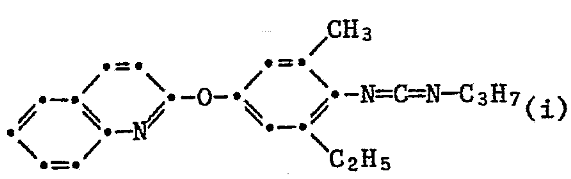

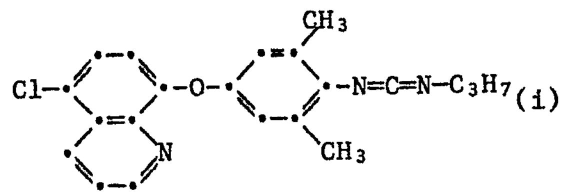

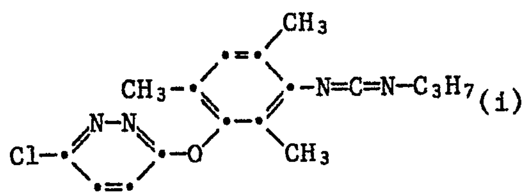

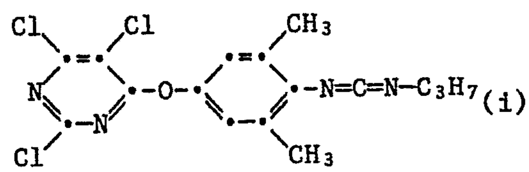

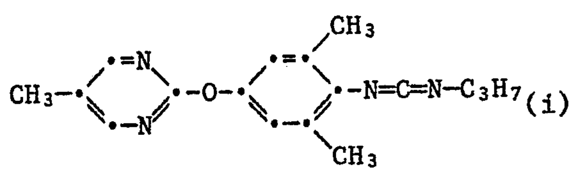

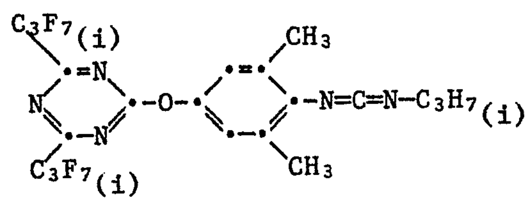

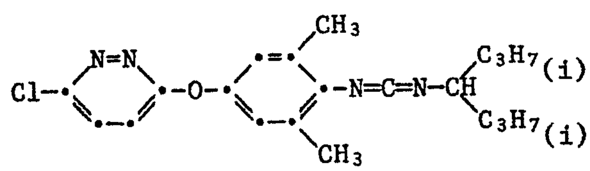

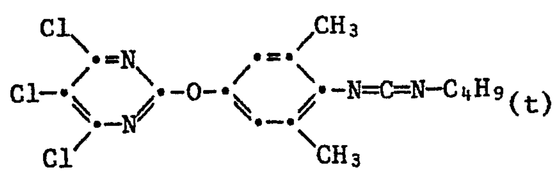

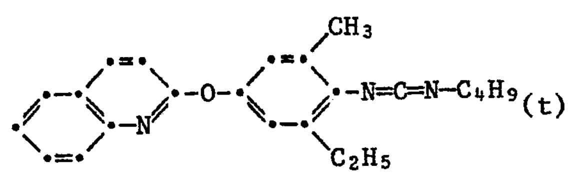

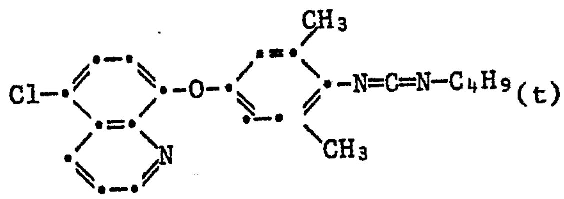

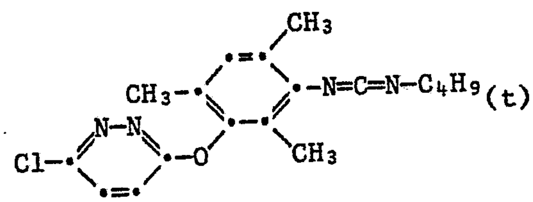

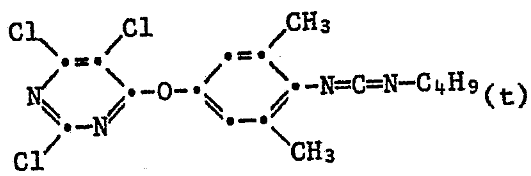

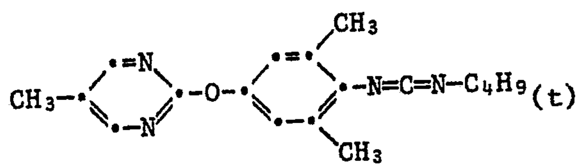

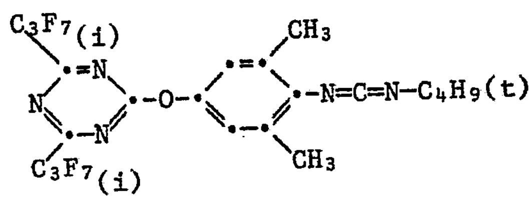

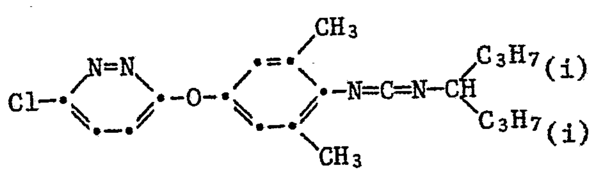

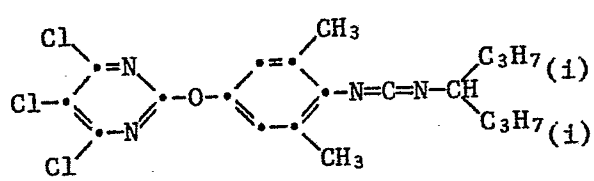

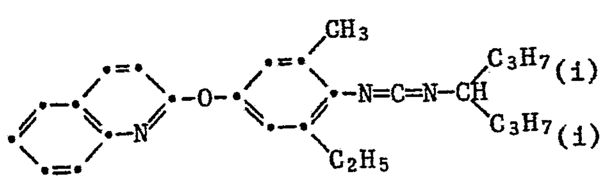

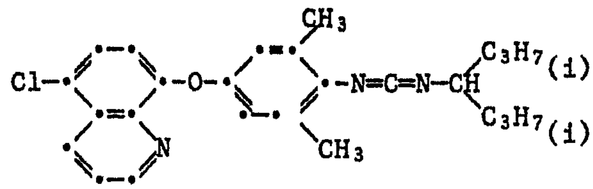

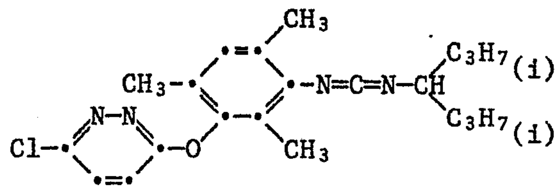

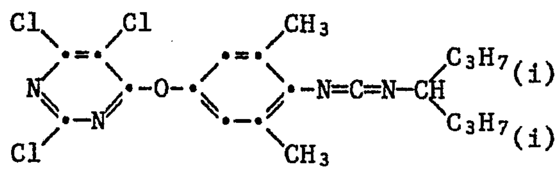

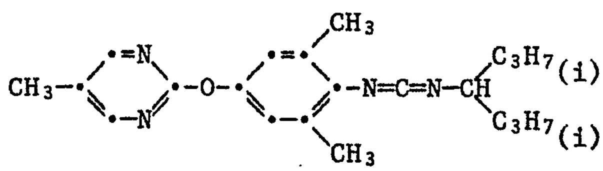

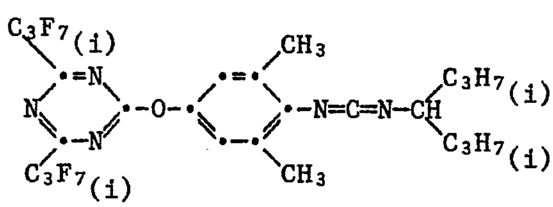

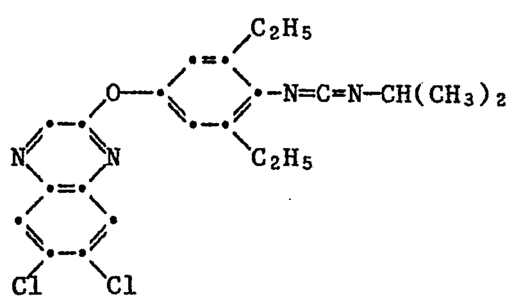

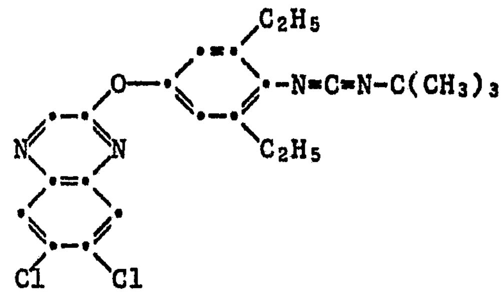

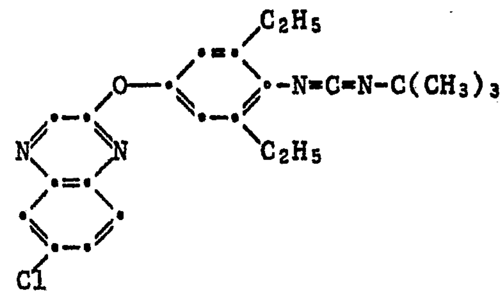

Beispiel 4: Formulierungen für flüssige Wirkstoffe der Formel I gemäss Beispiel 1 bis 3 oder Kombinationen dieser Wirkstoffe mit anderen Insektiziden oder Akariziden (% = Gewichtsprozent):

4.1. Emulsions-Konzentrate

| | a) | b) | c) |
|---|---|---|---|
| Wirkstoff resp. Wirkstoffkombination | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

4.2. Lösungen

| | a) | b) | c) | d) |
|---|---|---|---|---|
| Wirkstoff resp. Wirkstoffkombination | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxidiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

4.3. Granulate

| | a) | b) |
|---|---|---|
| Wirkstoff resp. Wirkstoffkombination | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

4.4. Stäubemittel

| | a) | b) |
|---|---|---|
| Wirkstoff resp. Wirkstoffkombination | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungen für feste Wirkstoffe der Formel I gemäss Beispiel 1 bis 3 oder Kombinationen dieser Wirkstoffe mit andern Insektiziden oder Akariziden (% = Gewichtsprozent):

4.5. Spritzpulver

| | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

4.6. Emulsions-Konzentrat

| | |
|---|---|
| Wirkstoff oder Wirkstoffkombination | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

4.7. Stäubemittel

| | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4.8. Extruder-Granulat

| | |
|---|---|
| Wirkstoff oder Wirkstoffkombination | 10 % |
| Na-Ligninsulfonat | 2 % |
| Carboxymethylcellulose | 1 % |
| Kaolin | 87 % |

Der Wirkstoff oder die Wirkstoffkombination wird mit den Zusatzstoffen vermischt, vermahlen und mit Wasser angefeuchtet. Dieses Gemisch wird extrudiert, granuliert und anschliessend im Luftstrom getrocknet.

4.9. Umhüllungs-Granulat

| | |
|---|---|
| Wirkstoff oder Wirkstoffkombination | 3 % |
| Polyäthylenglykol (MG 200) | 3 % |
| Kaolin | 94 % |

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

4.10. Suspensions-Konzentrat

| | |
|---|---|
| Wirkstoff oder Wirkstoffkombination | 40 % |
| Aethylenglykol | 10 % |

| | |
|---|---|
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 5: Wirkung gegen Musca domestica

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 bis 3 zeigen gute Wirkung im obigen Test.

Beispiel 6: Wirkung gegen Lucilia sericata

Zu 9 ml eines Zuchtmediums wird 1 ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 bis 3 zeigen in diesem Test gute Wirkung gegen Lucilia sericata.

Beispiel 7: Wirkung gegen Aëdes aegypti

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach Verdunsten des Acetons wird der Behälter mit 30 bis 40 2-tägigen Aëdes-Larven beschickt. Nach 2 und 7 Tagen wird die Mortalität geprüft.

Verbindungen der Formel I gemäss Beispiel 1 bis 3 zeigen gute Wirkung im obigen Test.

Beispiel 8: Insektizide Frassgift-Wirkung

Baumwollpflanzen (ca. 20 cm hoch) werden mit wässrigen Wirkstoffemulsionen (erhalten aus einem 10%igen emulgierbaren Konzentrat) besprüht, wobei die Wirkstoffemulsionen 400 ppm der jeweils zu prüfenden Verbindung enthalten.

Nach dem Antrocknen des Belages werden die Baumwollpflanzen mit Spodoptera littoralis- und Heliothis virescens-Larven im dritten larvalen Stadium besetzt. Der Versuch wird bei 24°C und 60 % relativer Luftfeuchtigkeit durchgeführt. In Abständen von jeweils 24 Stunden werden Mortalität sowie Entwicklungs- und Häutungsstörungen der angesetzten Larven bestimmt.

Verbindungen der Formel I gemäss Beispiel 1 bis 3 zeigen gute Wirkung im obigen Test.

Beispiel 9: Wirkung auf Spodoptera littoralis und Heliothis virescens (Larven und Eier)

Es werden drei in Töpfen gezogene Baumwollpflanzen von ca. 15-20 cm Höhe mit einer sprühfähigen flüssigen Zubereitung des zu prüfenden Wirkstoffes in einer Konzentration von 800 ppm behandelt. Nach Antrocknen des Sprühbelages werden die eingetopften Pflanzen in ein Blechgefäss von etwa 20 Litern Inhalt gestellt, das mit einer Glasplatte abgedeckt wird. Die Feuchtigkeit im Innern des abgedeckten Gefässes wird so reguliert, dass sich kein Kondenswasser bilden kann. Direktes, auf die Pflanzen fallendes Licht wird vermieden. Dann werden die drei Pflanzen infestiert, und zwar insgesamt mit:

a) 50 Larven von Spodoptera littoralis oder Heliothis virescens des ersten larvalen Stadiums;
b) 20 Larven von Spodoptera littoralis oder Heliothis virescens des dritten larvalen Stadiums;
c) zwei Eispiegeln von Spodoptera littoralis oder Heliothis virescens (dazu werden je 2 Blätter einer der Baumwoll-Pflanzen in einem beidseitig mit Gaze verschlossenen Plexiglaszylinder eingeschlossen; zwei Eispiegel von Spodoptera oder ein Teil eines Baumwollblattes mit darauf abgelegten Eiern von Heliothis werden zu den eingeschlossenen Blättern gegeben).

Nach 4 und 5 Tagen erfolgt die Auswertung gegenüber unbehandelten Kontrollen unter Berücksichtigung folgender Kriterien:

a) Anzahl der noch lebenden Larven,
b) larvale Entwicklungs- und Häutungsstörungen,
c) Frassschaden (Schabfrass und Lochfrass),
d) Schlupfrate (Anzahl der aus den Eiern geschlüpften Larven).

Verbindungen der Formel I gemäss Beispiel 1 bis 3 zeigen gute Gesamt-Wirksamkeit in obigem Test.

Beispiel 10: Ovizide Wirkung auf Spodoptera littoralis
Auf Filterpapier abgelegte Eier von Spodoptera littoralis werden aus dem Papier ausgeschnitten und in eine Lösung von 800 ppm des Wirkstoffes in einem Aceton-Wasser-Gemisch (1:1) getaucht. Die so behandelten Eiablagen werden dann aus diesem Gemisch herausgenommen und bei 28°C und 60 % relativer Feuchtigkeit in Kunststoffschalen deponiert.

Nach 5 Tagen wird die Schlupfrate, d.h. die Anzahl Larven, die sich aus den behandelten Eiern entwickelt haben, bestimmt.

Verbindungen der Formel I gemäss Beispiel 1 bis 3 zeigen gute Wirkung im obigen Test.

Beispiel 11: Ovizide Wirkung auf Laspeyresia pomonella
Abgelegte Eier von Laspeyresia pomonella, die nicht älter als 24 Stunden sind, werden auf Filterpapier für 1 Minute in eine acetonisch-wässrige Lösung enthaltend 800 ppm des zu prüfenden Wirkstoffes eingetaucht. Nach dem Antrocknen der Lösung werden die Eier in Petrischalen ausgelegt und bei einer Temperatur von 28°C belassen. Nach 6 Tagen wird der prozentuale Schlupf aus den behandelten Eiern bewertet.

Verbindungen der Formel I gemäss Beispiel 1 bis 3 zeigen gute Wirkung im obigen Test.

Beispiel 12: Wirkung gegen Anthonomus grandis (Adulte)
Zwei eingetopfte Baumwollpflanzen im 6-Blattstadium werden mit wässrigen benetzungsfähigen Emulsions-Zubereitungen, enthaltend 400 ppm des zu prüfenden Wirkstoffes besprüht. Nach dem Antrocknen des Spritzbelages (etwa 1,5 Stunden) wird jede Pflanze mit 10 adulten Käfern (Anthonomus grandis) besiedelt. Plastikzylinder, deren obere Oeffnungen mit Gaze abgedeckt sind, werden dann über die behandelten, mit den Testtieren besiedelten Pflanzen gestülpt, um ein Abwandern der Käfer zu verhindern. Die behandelten Pflanzen

werden bei 25°C und etwa 60 % relativer Luftfeuchtigkeit gehalten. Die Auswertung erfolgt nach 2, 3, 4 und 5 Tagen unter Zugrundelegung der prozentualen Mortalität der eingesetzten Testtiere (% Rückenlage) sowie der Antifeedant-Wirkung gegenüber unbehandelten Kontrollansätzen.

Verbindungen der Formel I gemäss Beispiel 1 bis 3 zeigen gute Wirkung im obigen Test.

Beispiel 13: Wirkung gegen pflanzenschädigende Akariden: Tetranychus urticae (OP-sensible) und Tetranychus cinnabarinus (OP-tolerant).

Die Primärblätter von Phaseolus vulgaris-Pflanzen werden 16 Stunden vor dem Versuch auf akarizide Wirkung mit einem infestierten Blattstück aus einer Massenzucht von Tetranychus urticae (OP-sens.) oder Tetranchus cinnabarinus (OP-tol.) belegt (Die Toleranz bezieht sich auf die Verträglichkeit gegenüber Diazinon).

Die so behandelten infestierten Pflanzen werden mit einer Versuchslösung enthaltend 800 ppm der jeweils zu prüfenden Verbindung bis zur Tropfnässe besprüht.

Nach 24 Stunden und wiederum nach 7 Tagen werden Imagines und Larven (alle beweglichen Stadien) unter dem Binokular auf lebende und tote Individuen ausgewertet. Während des Versuchsverlaufs stehen die Pflanzen in Gewächshauskabinen bei 25°C.

Verbindungen der Formel I gemäss Beispiel 1 bis 3 zeigen in diesem Versuch gute Wirkung.

Beispiel 14: Insektizide Kontaktwirkung: Myzus persicae

Etwa 4 cm hohe, in Wasser angezogene Erbsenkeimlinge werden vor Versuchsbeginn je mit ca. 200 Individuen der Spezies Myzus persicae besiedelt. Die so behandelten Pflanzen werden 24 Stunden später mit einer wässrigen Suspension enthaltend 800 ppm der zu prüfenden Verbindung bis zur Tropfnässe besprüht. Eine Auswertung der erzielten Abtötungsrate erfolgt 48 Stunden nach Applikation. Der Versuch wird bei 20-22°C und 60 % relativer Luftfeuchtigkeit durchgeführt.

Verbindungen der Formel I gemäss Beispiel 1 bis 3 zeigen gute Wirkung im obigen Test.

Beispiel 15: Insektizide Kontaktwirkung: Aphis craccivora

In Töpfen angezogene Pflanzen (Vicia faba) werden vor Versuchsbeginn mit je ca. 200 Individuen der Spezies Aphis craccivora besiedelt. Die so behandelten Pflanzen werden 24 Stunden später mit einer wässrigen Zubereitung enthaltend 400 ppm der zu prüfenden Verbindung bis zur Tropfnässe besprüht. Die Auswertung der erzielten Abtötungsrate erfolgt nach weiteren 24 Stunden.

Verbindungen der Formel I gemäss Beispiel 1 bis 3 zeigen gute Wirkung im obigen Test.

Beispiel 16: Wirkung gegen Laodelphax striatellus und Nilaparvata lugens (Nymphen)

Der Test wird an wachsenden Pflanzen durchgeführt. Dazu werden jeweils 4 Reispflanzen (Dicke des Stengels ca. 8 mm) mit einer Höhe von ca. 20 cm in Töpfe (Durchmesser von 8 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder

gestülpt und dieser mit einem Gaze-Deckel abgedeckt. Die Nymphen werden bis zum Erreichen des folgenden Entwicklungsstadiums über 10 Tage an den behandelten Pflanzen gehalten. Die Auswertung auf %-Mortalität erfolgt 1, 4 und 8 Tage nach der Behandlung.

Verbindungen der Formel I gemäss Beispiel 1 bis 3 zeigen gute Wirkung im obigen Test.

Beispiel 17: Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einen oberen Durchmeser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22°C gehalten. Zehn Tage nach dem Ansatz wird die in den Bechern enthaltene Erde abgesiebt und die zurückbleibenden Larven werden hinsichtlich ihrer Mortalität geprüft.

Verbindungen der Formel I gemäss Beispiel 1 bis 3 zeigen gute Wirkung im obigen Test.

Beispiel 18: Wirkung gegen Panonychus ulmi (OP und Carbamat resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfnässe besprüht. Die behandelten Pflanzen werden dann während weiteren 14 Tagen bei 25°C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen der Formel I gemäss Beispiel 1 bis 3 zeigen gute Wirkung im obigen Test.

Patentansprüche:

1. Verbindung der Formel I

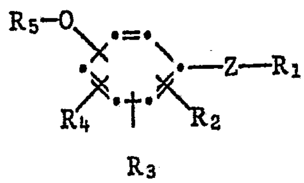 (I),

worin

$R_1$ $C_1$-$C_{12}$-Alkyl, $C_3$-$C_8$-Cycloalkyl, polycyclisches Alkyl mit insgesamt 7 bis 10 C-Atomen, mit 1 bis 12 Halogenatomen substituiertes $C_1$-$C_{12}$-Alkyl, mit 1 oder 2 $C_3$-$C_6$-Cycloalkylresten substituiertes $C_1$-$C_4$-Alkyl Alkoxyalkyl mit insgesamt 3 bis 10 C-Atomen, mit einem Phenylrest substituiertes $C_1$-$C_5$-Alkyl, mit einem ein- oder zweifach mit Halogen, Methyl, Methoxy oder Aethoxy substituierten Phenylrest substituiertes $C_1$-$C_5$-Alkyl, $C_3$-$C_{10}$-Alkenyl oder $C_3$-$C_{10}$-Alkinyl;

$R_2$ Wasserstoff oder $C_1$-$C_4$-Alkyl;

$R_3$ $C_1$-$C_4$-Alkyl;

$R_4$ Wasserstoff oder $C_1$-$C_4$-Alkyl; und

$R_5$ einen der Reste

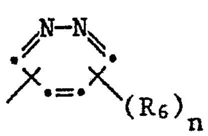 , 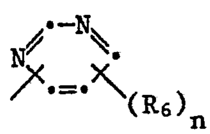 , 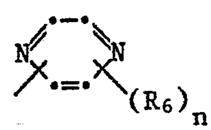 ,

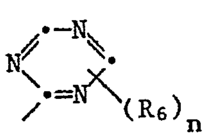 , 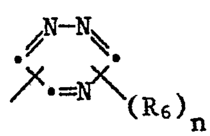 , 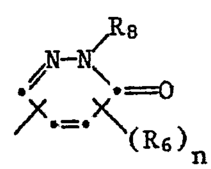 ,

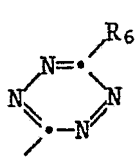 , 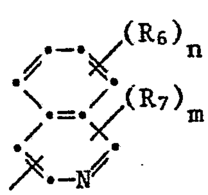 , 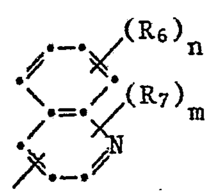 ,

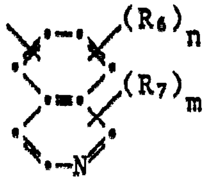 , 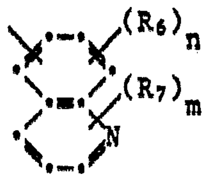 , 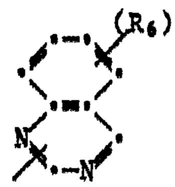 , 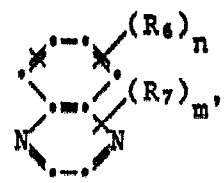

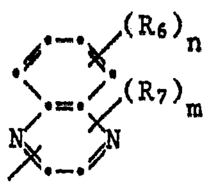 , 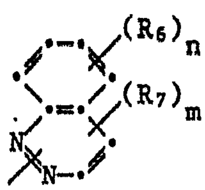 oder 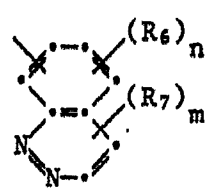 ;

$R_6$ und $R_7$ unabhängig voneinander Halogen, $C_1$-$C_4$-Alkyl, mit 1 bis 7 Halogenatomen substituiertes $C_1$-$C_3$-Alkyl, $C_1$-$C_4$-Alkoxy, mit 1 bis 7 Halogenatomen substituiertes $C_1$-$C_3$-Alkoxy oder Phenyl;

$R_8$ $C_1$-$C_4$-Alkyl;

$R_9$ $C_1$-$C_4$-Alkyl oder $C_3$-$C_4$-Alkenyl;

n eine ganze Zahl von 0 bis 3;

m eine ganze Zahl von 0 bis 2; und

Z eine der Gruppen $-NH-\overset{S}{\overset{\|}{C}}-NH-$, $-N=\overset{SR_9}{\overset{|}{C}}-NH-$ oder $-N=C=N-$

bedeuten, sowie deren Salze.

2. Verbindung gemäss Anspruch 1 der Formel Ia

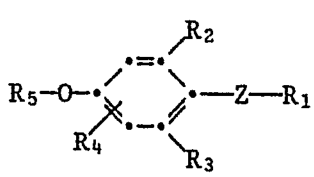 (Ia),

wobei die Reste $R_1$ bis $R_5$ und Z die in Anspruch 1 angegebene Bedeutungen haben.

3. Verbindung gemäss Anspruch 1 oder 2, dadurch gekennzeichnet, dass $R_4$ Wasserstoff bedeutet.

4. Verbindung gemäss einem der Ansprüche 1 bis 3, dadurch gekennzeichnet, dass Z die Gruppe $-NH-\overset{S}{\overset{\|}{C}}-NH-$ bedeutet.

5. Verbindung gemäss einem der Ansprüche 1 bis 3, dadurch gekennzeichnet, dass Z die Gruppe 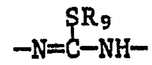 bedeutet.

6. Verbindung gemäss einem der Ansprüche 1 bis 3, dadurch gekennzeichnet, dass Z die Gruppe $-N=C=N-$ bedeutet.

7. Verbindung gemäss einem der Ansprüche 1 bis 6, dadurch gekennzeichnet, dass $R_5$ einen der Reste

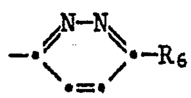 , 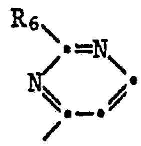 , 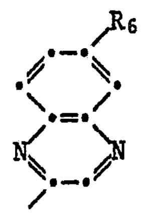 oder 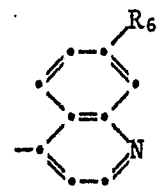

bedeutet, wobei $R_6$ für Halogen oder Trifluormethyl steht.

8. Verbindung gemäss einem der Ansprüche 1 bis 7, dadurch gekennzeichnet, dass $R_1$ $C_3$-$C_5$-Alkyl, $R_2$ und $R_3$ unabhängig voneinander $C_1$-$C_3$-Alkyl und $R_9$ Methyl oder Aethyl bedeuten.

9. Verbindungen gemäss einem der Ansprüche 1 bis 7, dadurch gekennzeichnet, dass $R_1$ Isopropyl oder tert.-Butyl, $R_2$ und $R_3$ Methyl oder Aethyl, $R_4$ Wasserstoff und $R_9$ Methyl bedeuten.

10. Verbindung gemäss Anspruch 4 der Formel

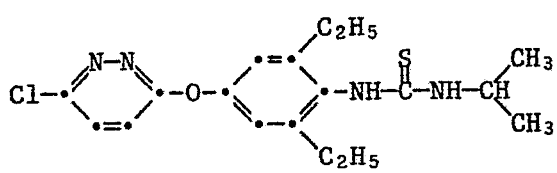 .

11. Verbindung gemäss Anspruch 4 der Formel

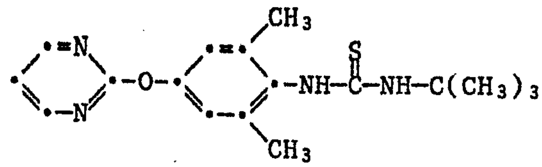
.

12. Verbindung gemäss Anspruch 4 der Formel

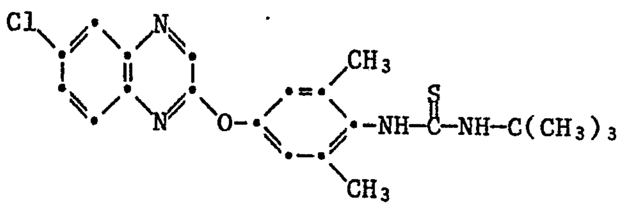
.

13. Verbindung gemäss Anspruch 4 der Formel

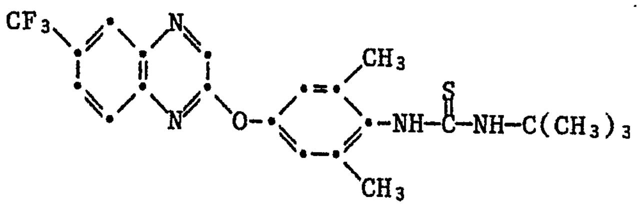
.

14. Verbindung gemäss Anspruch 5 der Formel

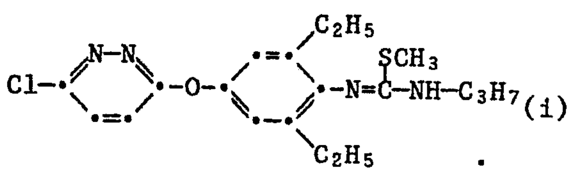
.

15. Verbindung gemäss Anspruch 5 der Formel

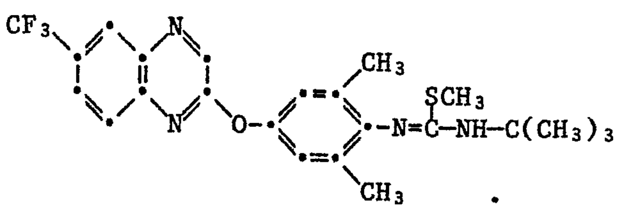
.

16. Verbindung gemäss Anspruch 5 der Formel

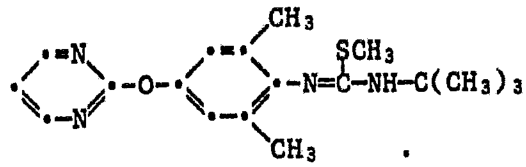

.

17. Verbindung gemäss Anspruch 6 der Formel

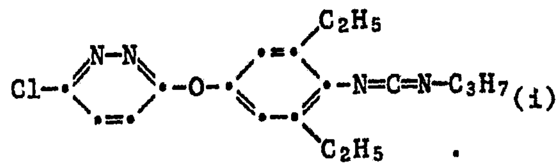

.

18. Verbindung gemäss Anspruch 6 der Formel

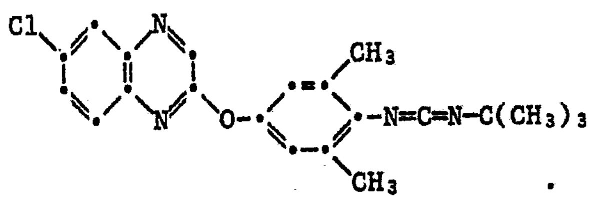

.

19. Verfahren zur Herstellung einer Verbindung gemäss Anspruch 1 der Formel I, worin Z die Gruppe $-NH-\overset{S}{\overset{\|}{C}}-NH-$ bedeutet, dadurch gekennzeichnet, dass man eine Verbindung der Formel II

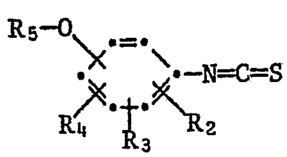

(II)

mit einer Verbindung der Formel III

$R_1-NH_2$ (III)

umsetzt, wobei die Reste $R_1$ bis $R_6$ und n die unter Anspruch 1 angegebenen Bedeutungen haben.

20. Verfahren zur Herstellung einer Verbindung gemäss Anspruch 1

der Formel I, worin Z die Gruppe $-N{=}\overset{SR_9}{C}-NH-$ bedeutet, oder eines ihrer Salze, dadurch gekennzeichnet, dass man eine Verbindung der Formel I, worin Z die Gruppe $-NH-\overset{S}{\overset{\|}{C}}-NH-$ bedeutet, mit einer Verbindung der Formel IV

$R_9$-X (IV)

umsetzt, wobei der Rest $R_3$ die unter Anspruch 1 angegebene Bedeutung hat und X eine Abgangsgruppe darstellt, und dass man gewünschtenfalls eine erhaltene Verbindung der Formel I in eines ihrer Salze überführt.

21. Verfahren zur Herstellung einer Verbindung gemäss Anspruch 1 der Formel I, worin Z die Gruppe $-N{=}C{=}N-$ bedeutet, dadurch gekennzeichnet, dass man aus einer Verbindung der Formel I, worin Z die Gruppe $-NH-\overset{S}{\overset{\|}{C}}-NH-$ bedeutet, Schwefelwasserstoff abspaltet.

22. Verbindung der Formel II gemäss Anspruch 19.

23. Verfahren zur Herstellung einer Verbindung der Formel II gemäss Anspruch 22, dadurch gekennzeichnet, dass man eine Verbindung der Formel V

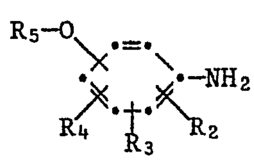

(V)

mit Thiophosgen umsetzt, wobei $R_2$ bis $R_5$ die unter Anspruch 1 angegebenen Bedeutungen haben.

24. Verbindung der Formel V gemäss Anspruch 23.

25. Schädlingsbekämpfungsmittel, welches als aktive Komponente eine Verbindung gemäss einem der Ansprüche 1 bis 18 zusammen mit geeigneten Trägern und/oder anderen Zuschlagstoffen enthält.

26. Verwendung einer Verbindung gemäss einem der der Ansprüche 1 bis 18 zur Bekämpfung von Insekten und Vertretern der Ordnung Akarina an Tieren und Pflanzen.

27. Verwendung gemäss Anspruch 26 zur Bekämpfung von larvalen Stadien pflanzenschädigender Insekten.

28. Verwendung nach Anspruch 26 zur Bekämpfung pflanzenschädigender Spinnmilben und fressender Insekten.

29. Verfahren zur Bekämpfung von Insekten und Vertretern der Ordnung Akarina, dadurch gekennzeichnet, dass man die Schädlinge bzw. deren verschiedene Entwicklungsstadien oder ihren Aufenthaltsort mit einer pestizid wirksamen Menge einer Verbindung der Formel I gemäss einem der Ansprüche 1 bis 18 oder mit einem Mittel enthaltend neben Zusatz- und Trägerstoffen eine pestizid wirksame Menge dieser Verbindung, in Kontakt bringt oder behandelt.

FO 7.5/EIC/cp*/ch*